# FASCICULE DE BREVET EUROPEEN

(11) **EP 0 974 336 B1**
(45) Date de publication et mention de la délivrance du brevet: **12.09.2001**
(21) Numéro de dépôt: 99401159.1
(22) Date de dépôt: 11.05.1999
(51) Int. Cl.: A61K 7/13

(54) **Composition de teinture d'oxidation des fibres kératiniques et procédé de teinture mettant en oeuvre cette composition**
Oxidationsfärbungszusammensetzung für keratinische Fäser und Färbungsverfahren mit derselben
Composition for oxidative dying of keratinous fibres and dying process therewith

(30) Priorité: 26.05.1998 FR 9806604
(43) Date de publication de la demande: 26.01.2000
(73) Titulaire: L'OREAL, 75008 Paris (FR)
(72) Inventeur: Lang, Gérard, 95390 Saint-Prix (FR); Cotteret, Jean, 78480 Verneuil sur Seine (FR)
(74) Mandataire: Goulard, Sophie

(56) Documents cités:
- EP-A- 0 850 636
- EP-A- 0 850 637
- EP-A- 0 850 638
- FR-A- 2 282 860
- US-A- 3 985 499

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, et au moins un colorant cationique particulier à titre de colorant direct, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant.

Ces colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La présente invention vise à proposer de nouvelles compositions pour la coloration d'oxydation des fibres kératiniques et en particulier des fibres kératiniques humaines telles que les cheveux qui permettent d'aboutir à des colorations riches en reflets tout en présentant de bonne propriétés de ténacité.

Ainsi, la demanderesse vient en effet de découvrir qu'il est possible d'obtenir de nouvelles teintures à la fois riches en reflets et tenaces en associant :
- au moins une base d'oxydation,
- au moins un colorant direct cationique de formule (I) définie ci-après.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- au moins un colorant direct cationique de formule (I) suivante :

   A―N=N―B (I)

   dans laquelle :
   A représente un groupement choisi parmi les structures A1 à A3 suivantes : 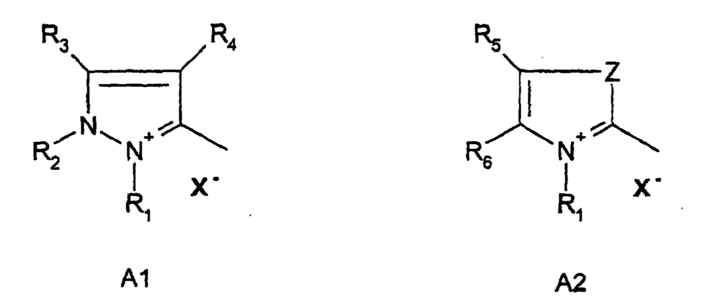 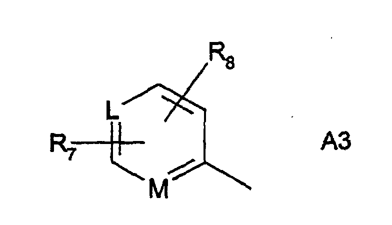 structures A1 à A3 dans lesquelles :
      - R₁ désigné un radical alkyle en C₁-C₄, un radical phényle ou un radical phényle substitué par un radical alkyle en C₁-C₄ ou par un atome d'halogène choisi parmi le chlore, le brome, l'iode et le fluor ;
      - R₂ désigne un radical alkyle en C₁-C₄ ou un radical phényle ;
      - R₃ et R₄, identiques ou différents, représentent un radical alkyle en C₁-C₄, un radical phényle, ou forment ensemble un cycle benzénique substitué par un ou plusieurs radicaux alkyle en C₁-C₄, alcoxy en C₁-C₄ ou nitro ; R₃ peut également désigner un atome d'hydrogène ;
      - R₅ et R₆, identiques ou différents, représentent un radical alkyle en C₁-C₄, un radical phényle, ou forment ensemble un cycle benzénique non substitué ou substitué par un ou plusieurs radicaux alkyle en C₁-C₄, alcoxy en C₁-C₄ ou nitro ; R₅ peut également désigner un atome d'hydrogène ;
      - R₇ et R₈, identiques ou différents, représentent un atome d'hydrogène ou d'halogène choisi parmi le chlore, le brome, l'iode et le fluor, un radical alkyle en C₁-C₄, alcoxy en C₁-C₄ ou un radical nitro ;
      - Z désigne un atome d'oxygène ou de soufre ou-un groupement NR₂ dans lequel R₂ peut prendre la même signification que celle indiquée ci-dessus ;
      - le sommet L représente un groupement -CH, -CR ou -N⁺R₉(X⁻)ᵣ;
      - le sommet M représente un groupement -CH, -CR ou -N⁺R₉(X⁻)ᵣ;
      - r est un nombre entier égal à 0 ou 1 ;
      - R représente un radical alkyle en C₁-C₄;
      - R₉ représente un atome O⁻, un radical alkyle en C₁-C₄ ou alcoxy en C₁-C₄;
      - X représente un anion de préférence choisi parmi le chlorure, le méthylsulfate, l'éthylsulfate, l'acétate et le perchlorate ;
      étant entendu que :
      - dans les groupements de formule A3, au moins un des sommets L et M représente un groupement -N⁺R₉(X⁻)ᵣ;
      - lorsque R₆ représente un radical alkyle en C₁-C₄ et que Z représente un atome de soufre, alors R₅ est différent d'un radical alkyle en C₁-C₄ ;
      - lorsque R₆ représente un radical alkyle en C₁-C₄ et que Z représente un atome de soufre, et que l'un des radicaux R₁₀ et R₁₁ représente un atome d'hydrogène, alors R₅ est différent d'un atome d'hydrogène ;
      - lorsque R₉ désigne O⁻, alors r = 0;
      - lorsque L ou M désigne radical un -N⁺R₉(X⁻)ᵣ dans lequel R₉ représente un radical alkyle en C₁-C₄ et r = 1, alors au moins un des radicaux R₇ et R₈ est différent d'un atome d'hydrogène ;
      - lorsque L désigne un radical -N⁺R₉(X⁻)ᵣ, alors M représente un groupement -CH ou CR ;
      - lorsque M désigne un radical -NR₉(X⁻)ᵣ, alors L représente un groupement -CH ou CR ;
      - lorsque Z représente un groupement NR₂ dans lequel R₂ représente un radical alkyle en C₁-C₄, alors au moins un des radicaux R₁, R₅ et R₆ est différent d'un radical alkyle en C₁-C₄;
      - lorsque Z représente un atome de soufre et que R₁ représente un radical alkyle en C₁-C₄, et que l'un des radicaux R₁₀ et R₁₁ représente un atome d'hydrogène, alors R₅ et R₆ ne peuvent pas former ensemble un cycle benzénique- non substitué ;
   B représente:
      **soit (a)** un groupement de structure B1 suivante : 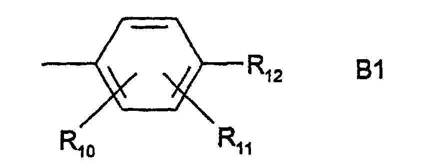 structure B1 dans laquelle,
      - R₁₀ représente un atome d'hydrogène, un atome d'halogène choisi parmi le chlore, le brome, l'iode et le fluor, un radical alkyle en C₁-C₄, alcoxy en C₁-C₄, hydroxyle, un groupement -NHR₁₃, -NR₁₄R₁₅, -NHCOalkyle en C₁-C₄, nitro, ou forme avec R₁₁ un cycle à 5 ou 6 chaînons carbonés ou contenant un ou plusieurs hétéroatomes choisis parmi l'azote, l'oxygène ou le soufre ;
      - R₁₁ représente un atome d'hydrogène, un atome d'halogène choisi parmi le chlore, le brome, l'iode et le fluor, un radical alkyle en C₁-C₄, alcoxy en C₁-C₄, ou forme avec R₁₂ ou R₁₃ un cycle à 5 ou 6 chaînons carbonés ou contenant un ou plusieurs hétéroatomes choisis parmi l'azote, l'oxygène ou le soufre ;
      - R₁₂ représente un atome d'hydrogène, un radical hydroxyle, un radical -NHR₁₃, ou un radical -NR₁₄R₁₅ ;
      - R₁₃ représente un atome d'hydrogène, un radical alkyle en C₁-C₄, monohydroxyalkyle en C₁-C₄, polyhydroxyalkyle en C₂-C₄ ou phényle ;
      - R₁₄ et R₁₅, identiques ou différents, représentent un radical alkyle en C₁-C₄, monohydroxyalkyle en C₁-C₄ ou polyhydroxyalkyle en C₂-C₄;
   **soit (b)** un groupement hétérocyclique azoté à 5 ou 6 chaînons, un groupement hétérocyclique azoté à 5 ou 6 chaînons renfermant un ou plusieurs hétéroatomes choisi parmi l'oxygène et le soufre et/ou un ou plusieurs groupements carbonylés, lesdits hétérocycles pouvant être substitués par un ou plusieurs radicaux alkyle en C₁-C₄, amino ou phényle.

Parmi lesdits groupements hétérocycles azotés à 5 ou 6 chaînons figurant ci-dessus, on peut notamment citer les groupements de structure B2 suivante : 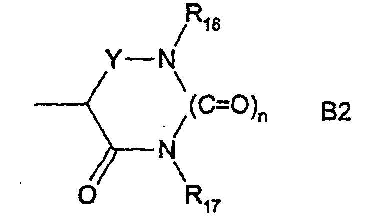 structure B2 dans laquelle :
- R₁₆ et R₁₇, identiques ou différents, représentent un atome d'hydrogène, un radical alkyle en C₁-C₄ ou un radical phényle ;
- Y représente un groupement 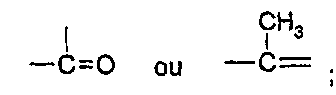 et
- n est un nombre entier égal à 0 ou 1.

Les colorants directs cationiques de formule (I) ci-dessus sont des composés connus de l'art antérieur et sont décrits par exemple dans la demande de brevet FR-A-2 140 205 et ses certificats d'addition, ainsi que dans la demande de brevet FR-A-2 189 006.

La composition tinctoriale conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux. Ces propriétés sont particulièrement remarquables en ce qui concerne la chromaticité.

L'invention a également pour objet un procédé de teinture d'oxydation des fibres kératiniques mettant en oeuvre cette composition tinctoriale.

Dans les composés de formule (I) ci-dessus, les radicaux alkyle et alcoxy en C₁-C₄ sont de préférence choisis parmi les radicaux méthyle, éthyle, butyle, méthoxy et éthoxy.

La nature de la ou des bases d'oxydation utilisées dans la composition tinctoriale conforme à l'invention n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (II) suivante et leurs sels d'addition avec un acide : 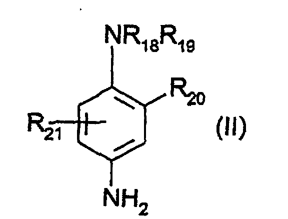 dans laquelle :
- R₁₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₁₉ représente un atome d'hydrogène, un radical alkyle en C₁-C₄, monohydroxyalkyle en C₁-C₄, polyhydroxyalkyle en C₂-C₄, alcoxy(C₁-C₄)alkyl(C₁-C₄) ou alkyle en C₁-C₄ substitué par un groupement azoté ;
- R₂₀ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₂₁ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium,

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.
Parmi les bases doubles utilisables à titre de bases d'oxydation dans les compositions tinctoriales conformes à l'invention, on peut notamment citer les composés répondant à la formule (III) suivante, et leurs sels d'addition avec un acide : 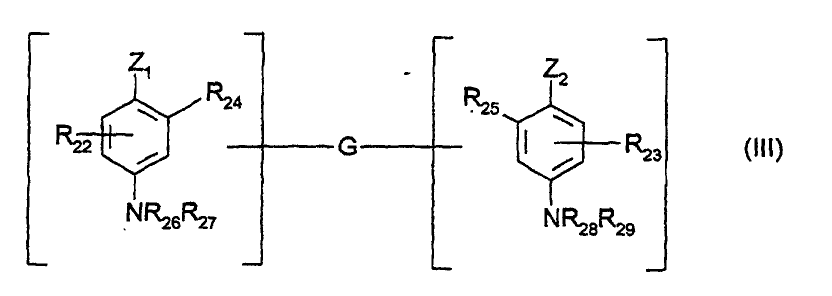 dans laquelle :
- Z₁ et Z₂, identiques ou différents, représentent un radical hydroxyle ou -NH₂ pouvant être substitué par un radical alkyle en C₁-C₄ ou par un bras de liaison G ;
- le bras de liaison G représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₂₂ et R₂₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison G ;
- R₂₄, R₂₅, R₂₆, R₂₇, R₂₈ et R₂₉, identiques ou différents, représentent un atome d'hydrogène, un bras de liaison G ou un radical alkyle en C₁-C₄ ;
étant entendu que les composés de formule (III) ne comportent qu'un seul bras de liaison G par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés de formule (IV) suivante, et leurs sels d'addition avec un acide : 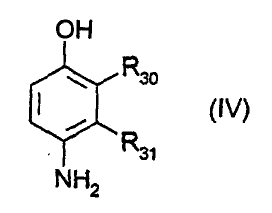 dans laquelle :
- R₃₀ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₃₁ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₃₀ et R₃₁ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 153 196, comme la 2,5-diamino pyridine, la 2-(4-méthoxyphényl)amino 3-amino pyridine, la 2,3-diamino 6-méthoxy pyridine, la 2-(p-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le ou les colorants directs cationiques de formule (I) conformes à l'invention sont de préférence choisis parmi :
- le diméthylamino-4'-phényl-azo-2-pyridine-N-oxyde de formule : 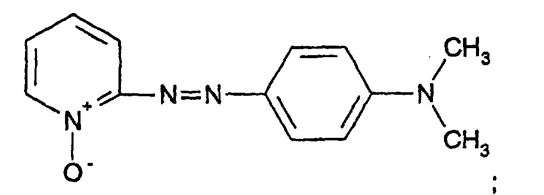
- le diméthylamino-4'-méthyl-2'-phényl-azo-2-pyridine-N-oxyde de formule : 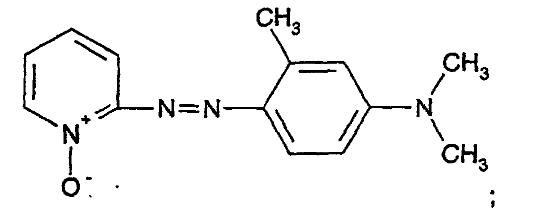
- l'iodure de 2-[(1,3-diamino-6-méthyl-phényl-4)-azo]-3-méthyl-benzothiazolium de formule : 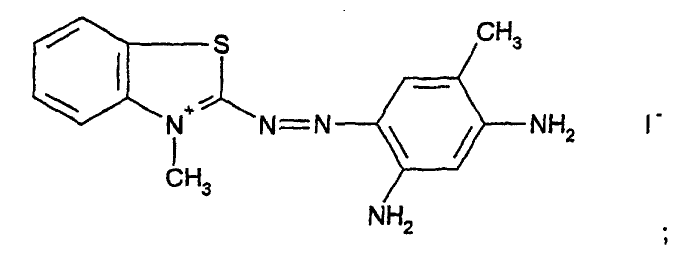
- le chlorure d'hydroxy-6' benzomorpholine-7'-aza-2-méthyl-3-benzothiazolium de formule : 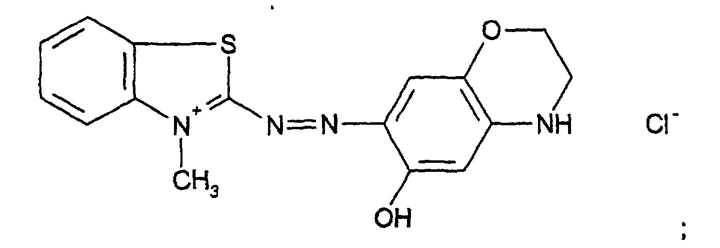
- le perchlorate d'hydroxy-6'-benzomorpholine-7'-aza-2-méthyl-4-phényl-3-thiazolium de formule : 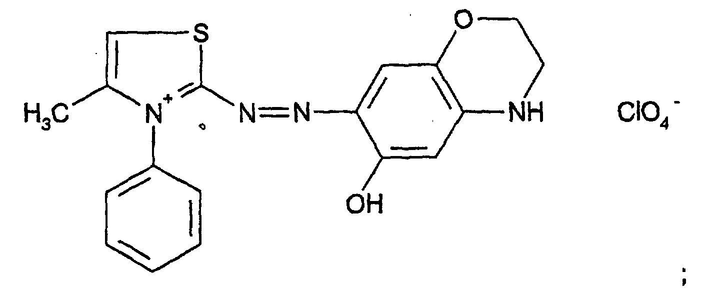
- le perchlorate d'hydroxy-6'-benzomorpholine-7'-azo-2-diphényl-3,4-thiazolium de formule : 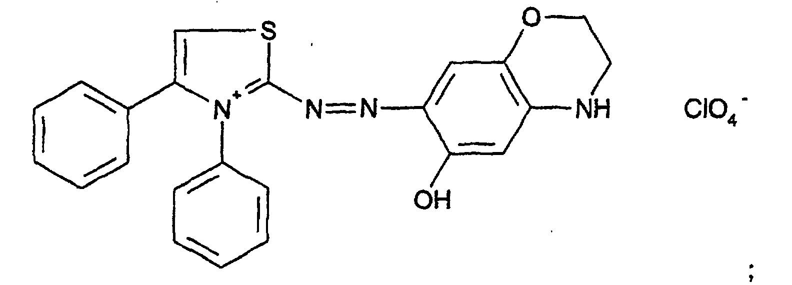
- le chlorure d'hydroxy-6'-benzomorpholine-7'-aza-2-méthyl-3-benzolthiazolium de formule : 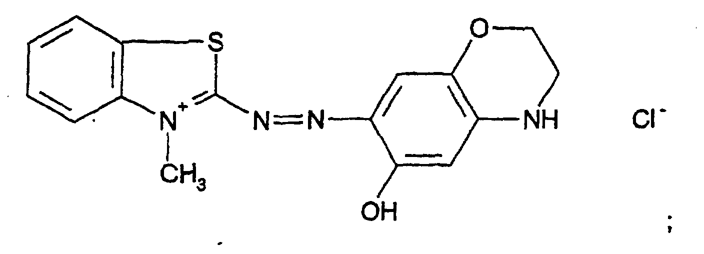
- le perchlorate d'hydroxy-6'-benzomorpholine-7'-aza-2-méthyl-4-phényl-3-thiazolium de formule : 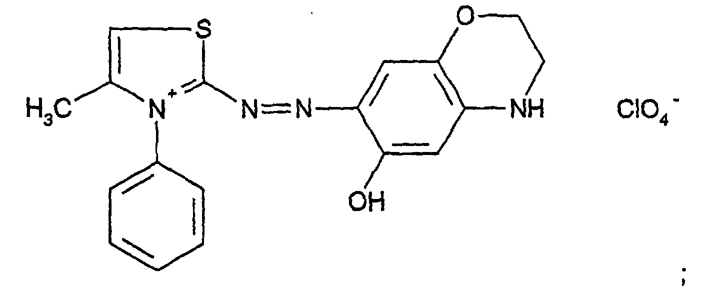
- le diamino-2',4'-méthoxy-5'-phényl-azo-2-pyridine-N-oxyde de formule : 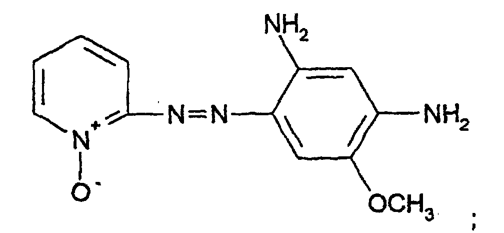
- l'acétamido-2'-hydroxy-4'-méthyl-5'-phényl-azo-2-pyridine N-oxyde de formule : 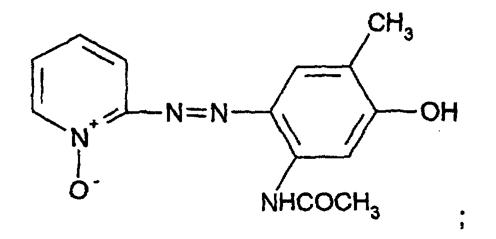
- l'acétamido-2'-diméthylamino-4'-phényl-azo-2-pyridine-N-oxyde de formule : 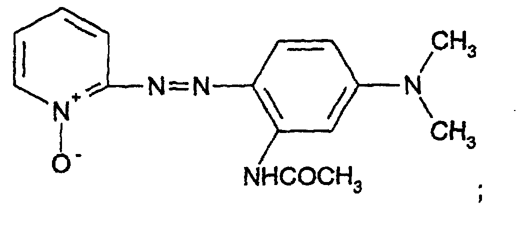
- l'amino-2'-diméthylamino-4'-phényl-azo-2-pyridine-N-oxyde de formule: 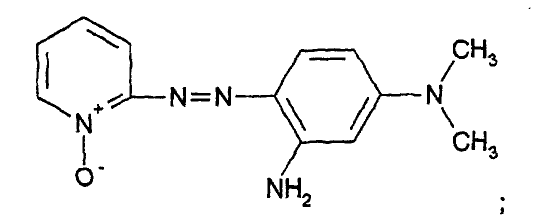
- le perchlorate de diamino-2',4'-méthyl-5'-phényl-azo-2-méthoxy-1-pyridinium de formule : 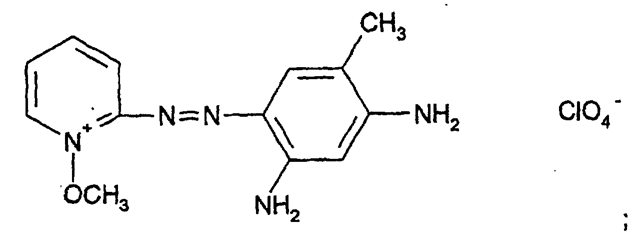
- le diméthyl-2',5'-hydroxy-4'-phényl-azo-2-pyridine-N-oxyde de formule : 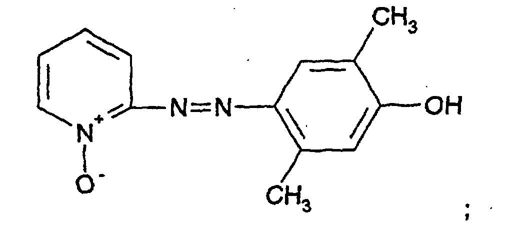
- le diméthylamino-2'-hydroxy-4'-phényl-azo-2-pyridine-N-oxyde de formule : 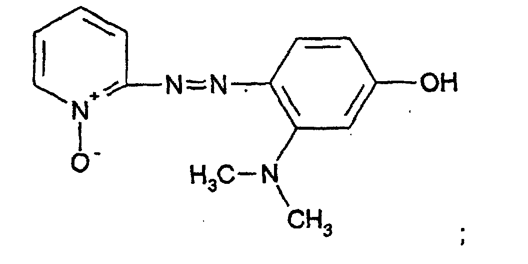
- le diméthylamino-4'-hydroxy-2'-phényl-azo-2-pyridine-N-oxyde de formule 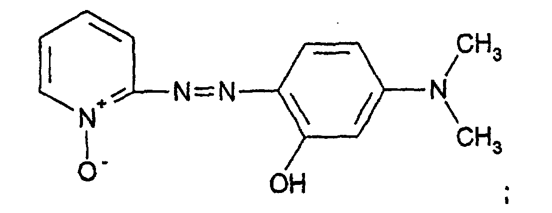
- le diméthyl-3',5'-hydroxy-4'-phényl-azo-2-pyridine-N-oxyde de formule : 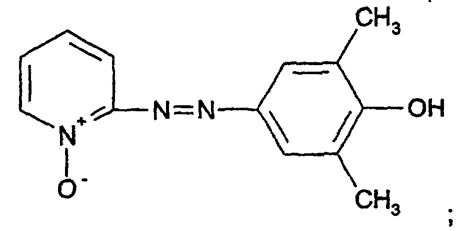
- le diméthylamino-4'-nitro-2'-phényl-azo-2-pyridine-N-oxyde de formule : 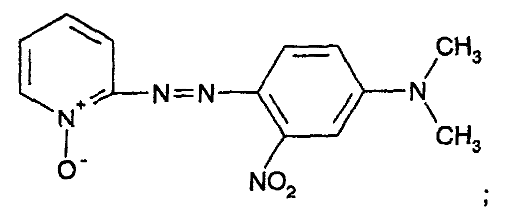
- l'hydroxy-8'-quinoléine-azo-5' : 2'-pyridine-N-oxyde de formule : 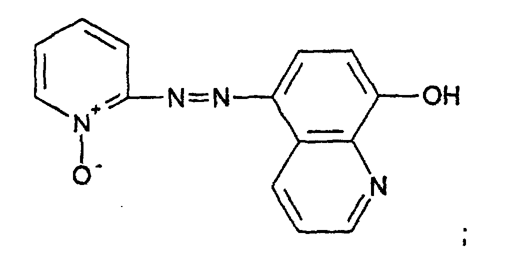
- le méthosulfate de nitro-2'-diméthylamino-4'-phényl-azo-2-méthoxy-1-pyridinium de formule : 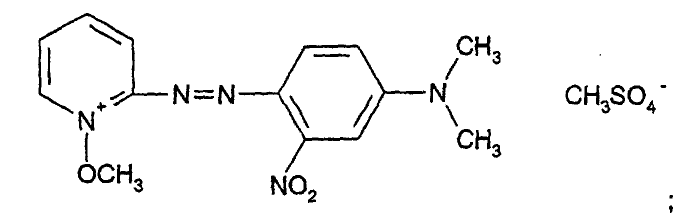
- le diméthylamino-4'-phényl-azo-2-nitro-5-pyridine-N-oxyde de formule : 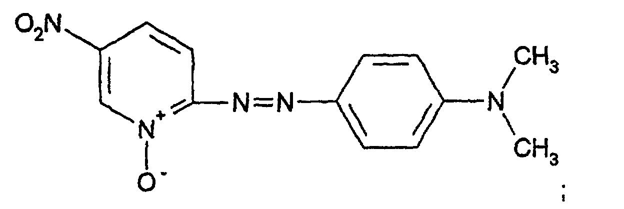
- le diméthylamino-4'-phényl-azo-2-méthyl-6-pyridine-N-oxyde de formule : 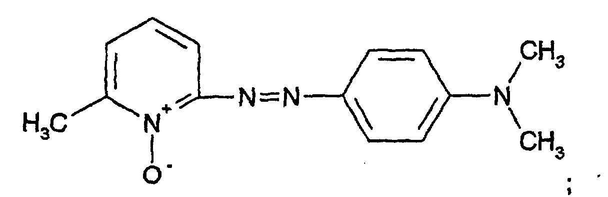
- le diméthylamino-4'-phényl-azo-2-méthyl-5-pyridine-N-oxyde de formule : 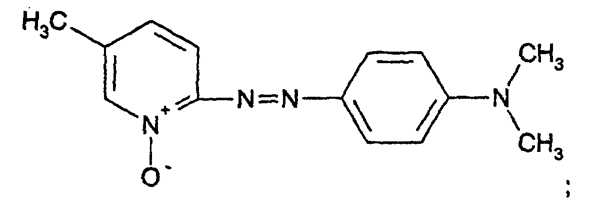
- le diméthylamino-4'-phényl-azo-2-méthyl-3-pyridine-N-oxyde de formule : 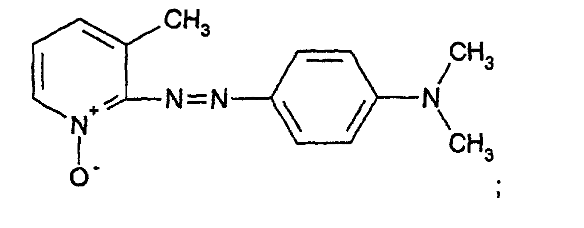
- le diéthylamino-4'-phényl-azo-2-méthyl-3-pyridine-N-oxyde de formule : 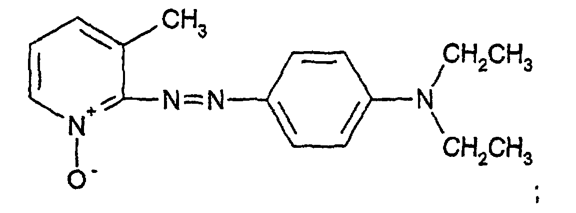
- le diméthylamino-4'-acétylamino-2'-phényl-azo-2-méthyl-3-pyridine-N-oxyde de formule : 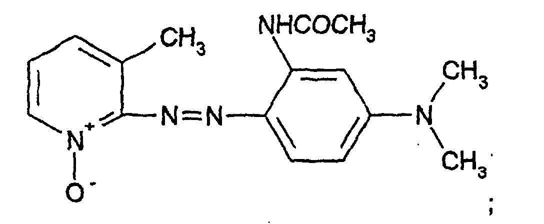
- l'amino-4'-naphtalène-azo-1' : 2-méthyl-6-pyridine-N-oxyde de formule : 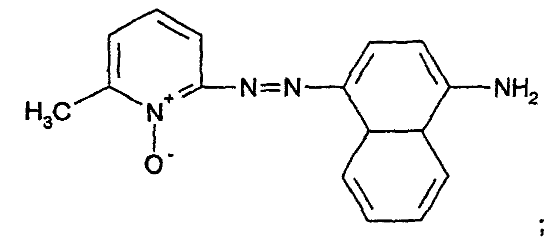
- le di-(β-hydroxyéthyl)amino-4'-phényl-azo-2-méthyl-6-pyridine-N-oxyde de formule : 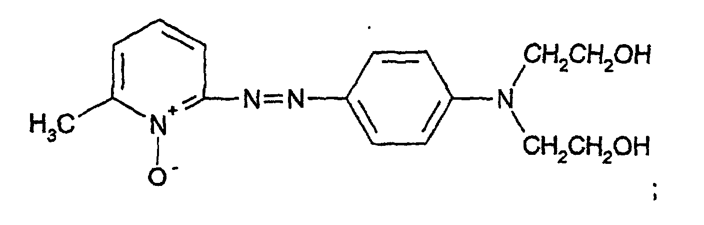
- le diéthylamino-4'-phényl-azo-2-méthyl-6-pyridine-N-oxyde de formule : 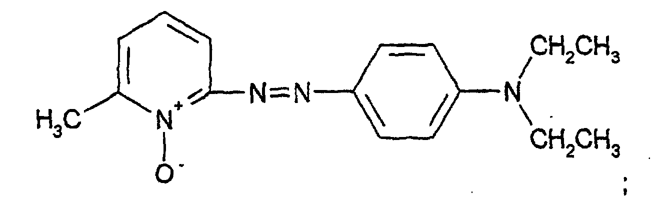
- le diméthyl-2',5'-hydroxy-4'-phényl-azo-2-méthyl-6-pyridine-N-oxyde de formule : 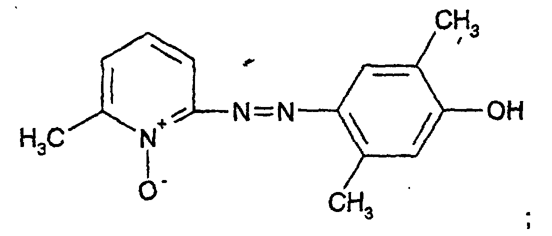
- le di-(β-hydroxyéthyl)amino-4'-phényl-azo-2-méthyl-4-pyridine-N-oxyde de formule : 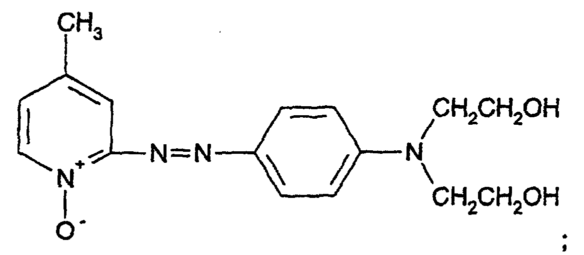
- le diéthylamino-4'-phényl-azo-2-méthyl-4-pyridine-N-oxyde de formule: 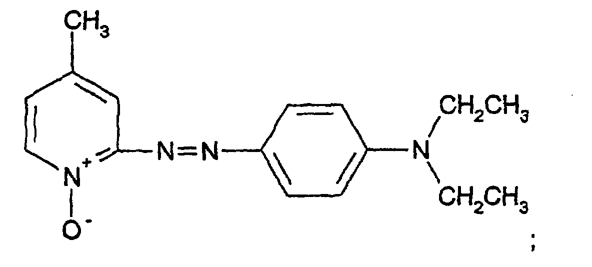
- l'amino-4'-naphtalène-azo-1' : 2-méthyl-4-pyridine-N-oxyde de formule : 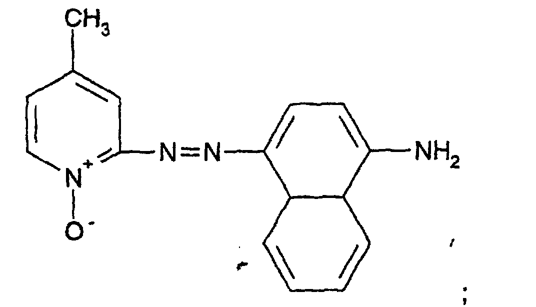
- le diamino-2',4'-méthyl-5'-phényl-azo-2-méthyl-4-pyridine-N-oxyde de formule : 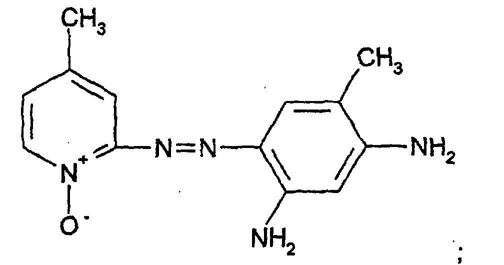
- le diméthylamino-4'-phényl-azo-2-méthyl-4-pyridine-N-oxyde de formule : 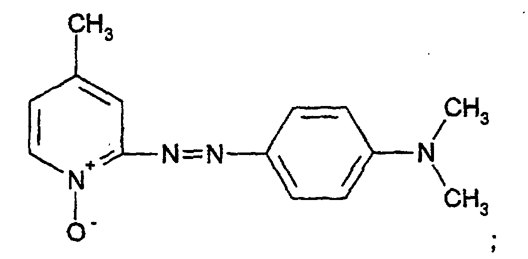
- le diméthylamino-4'-phényl-azo-2-diméthyl-4,6-pyridine-N-oxyde de formule : 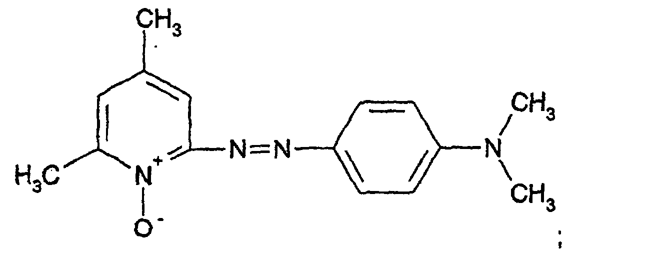
- le diméthyl-2',5'-hydroxy-4'-phényl-azo-2-méthyl-4-pyridine-N-oxyde de formule : 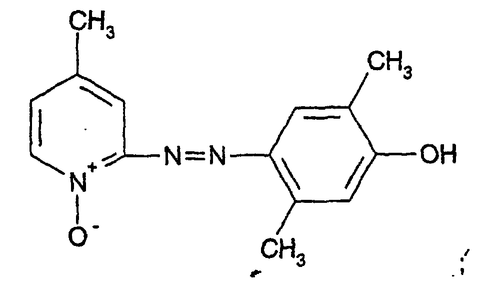
- le diméthylamino-4'-phényl-azo-2-chloro-5-pyridine-N-oxyde de formule : 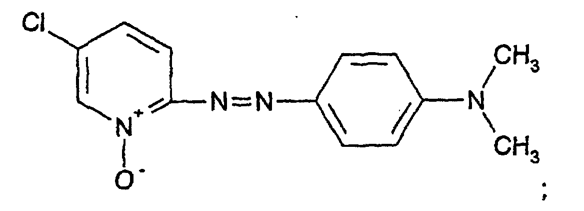
- le diméthylamino-4'-méthyl-2'-phényl-azo-2-chloro-5-pyridine-N-oxyde de formule: 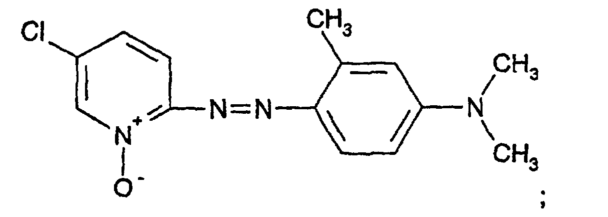
- le diéthylamino-4'-phényl-azo-2-chloro-5-pyridine-N-oxyde de formule : 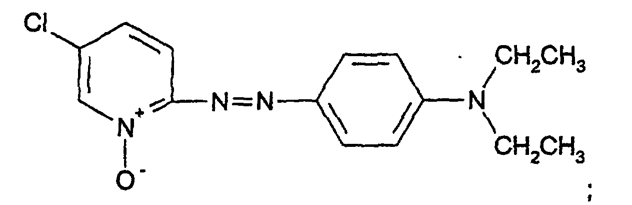
- l'hydroxy-6'-benzomorpholine-azo-7' : 2-pyridine-N-oxyde de formule : 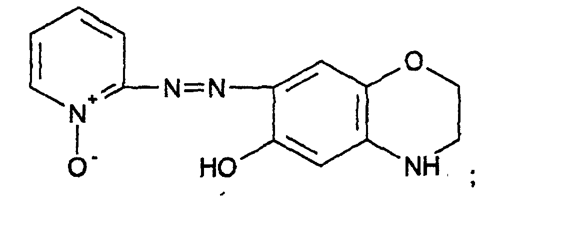
- le méthosulfate d'hydroxy-6'-benzomorpholine-azo-7' : 2-méthoxy-1-pyridinium de formule : 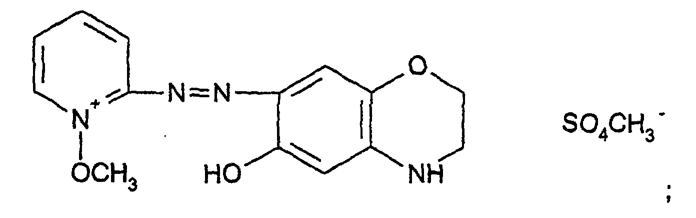
- le diamino-2',4'-méthyl-5'-phényl-azo-2-pyridine-N-oxyde de formule : 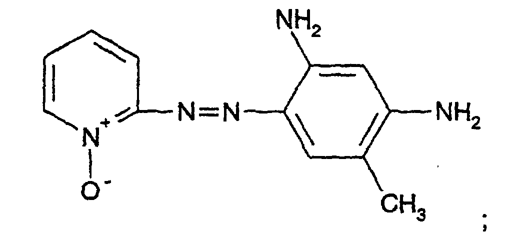
- le méthosulfate de méthyl-2'-oxo-5'-phényl-4'-dihydropyrazolyl-azo-2-méthoxy-1-pyridinium de formule : 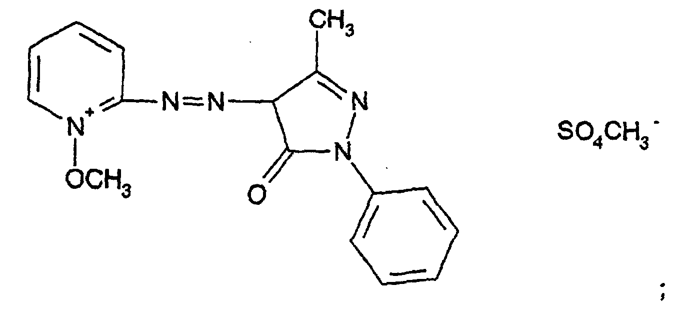
- le méthyl-3'-trioxo-2',4',6'-hexahydro-pyrimidinyl-azo-2-pyridine-N-oxyde de formule : 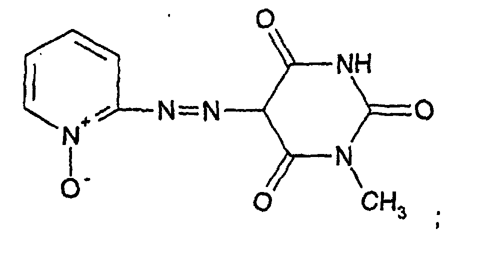
- l'amino-4'-phényl-azo-2-pyridine-N-oxyde de formule : 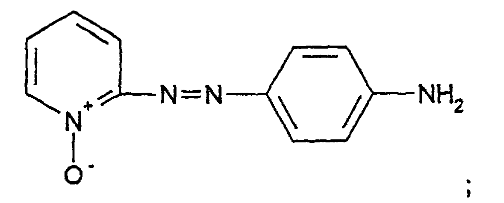
- le di-(β-hydroxyéthyl)amino-4'-phényl-azo-2-pyridine-N-oxyde de formule : 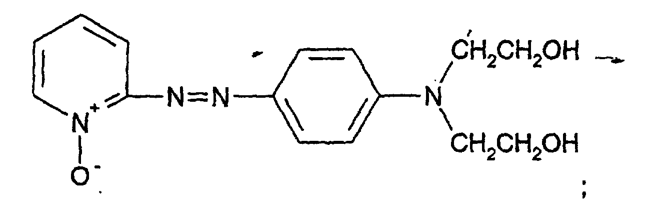
- le (N-phénylamino)-4'-phényl-azo-2-pyridine-N-oxyde de formule : 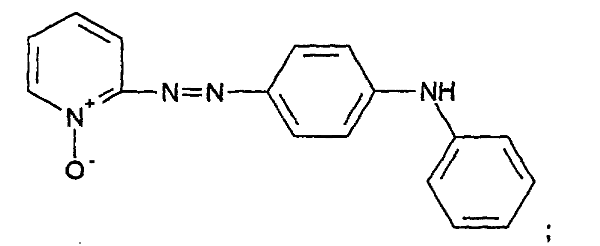
- le (N-phénylamino)-4'-phényl-azo-2-méthyl-3-pyridine-N-oxyde de formule : 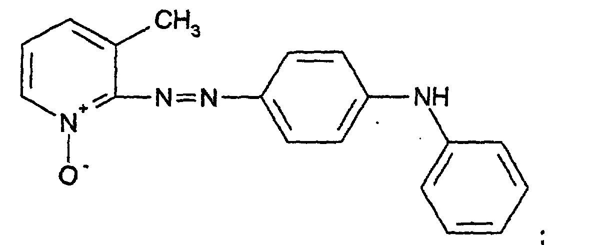
- le méthosulfate d'amino-4'-phényl-azo-2-diméthyl-1,3-pyridinium de formule : 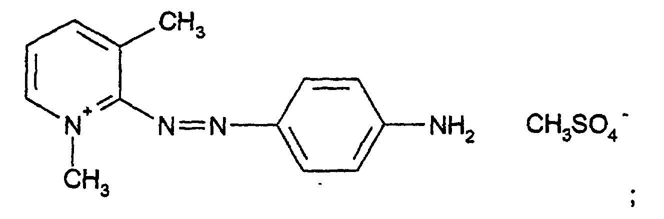
- le méthosulfate de di-(β-hydroxyéthyl)amino-4'-phényl-azo-2-diméthyl-1,4-pyridinium de formule : 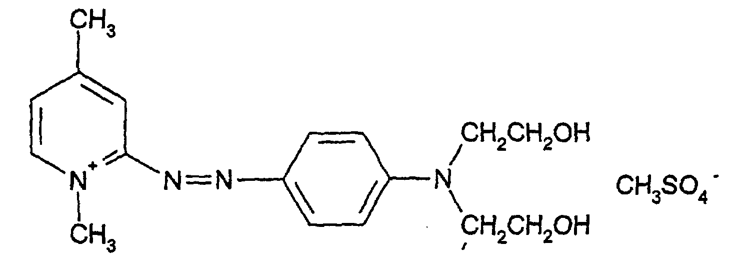
- le méthosulfate de diméthylamino-4'-phényl-azo-2-diméthyl-1,3-pyridinium de formule : 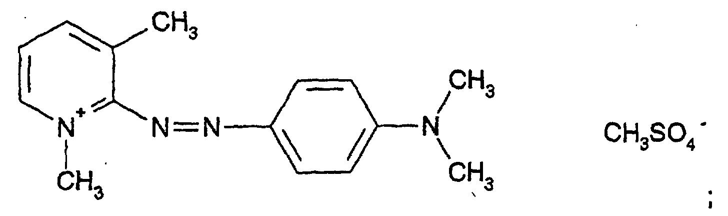
- le méthosulfate de N-phénylamino-4'-phényl-azo-2-diméthyl-1,6-pyridinium de formule : 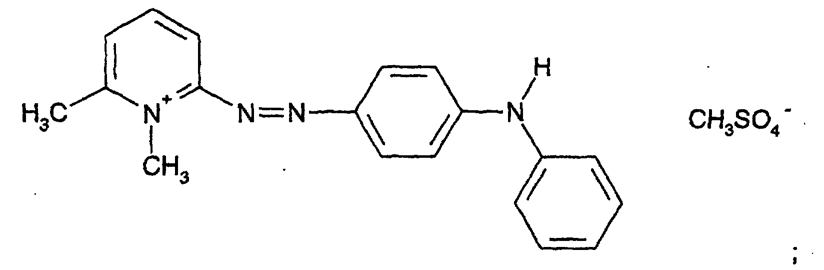
- le méthosulfate de diméthylamino-4'-phényl-azo-2-diméthyl-1,5-pyridinium de formule : 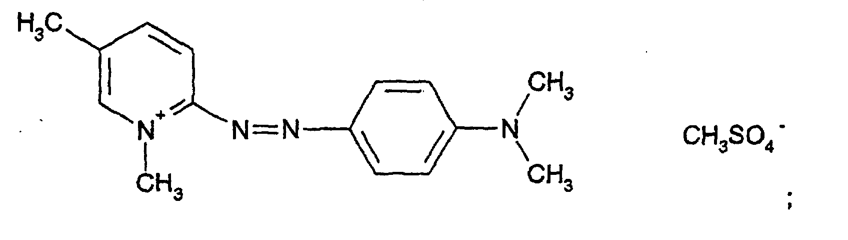
- le méthosulfate de diméthylamino-4'-phényl-azo-2-diméthyl-1,6-pyridinium de formule : 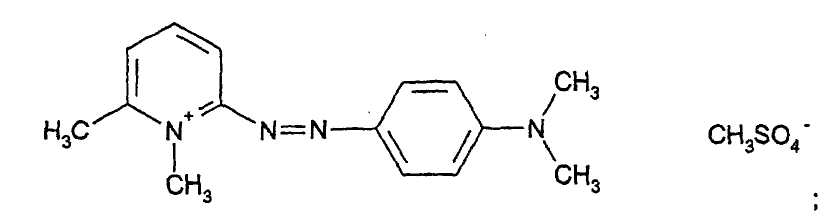
- le trioxo-2',4',6'-trioxo-hexahydro-pyrimidinyl-azo-2-pyridine-N-oxyde de formule : 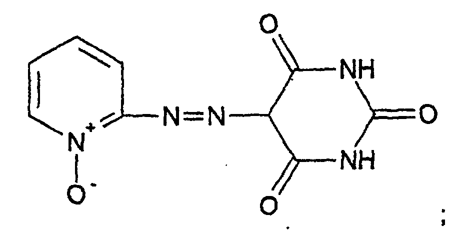
- le perchlorate de diméthylamino-4'-phényl-azo-2-diméthyl-1,3-benzimidazolium de formule : 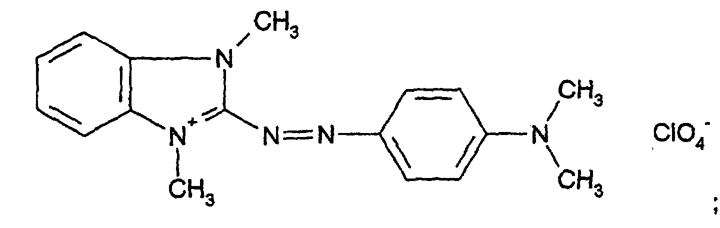 et
- le diamino-2',4'-pyridine-3'-azo-2-pyridine-N-oxyde de formule :

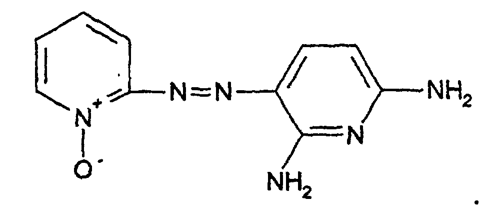

Le ou les colorants directs cationiques de formule (I) utilisés selon l'invention, représentent de préférence de 0,001 à 10 % en poids environ du poids total de la composition tinctoriale et encore plus préférentiellement de 0,01 à 5 % en poids environ de ce poids.

La composition tinctoriale conforme à l'invention peut en outre contenir un ou plusieurs coupleurs et/ou un ou plusieurs colorants directs additionnels différents des composés de formule (I), notamment pour modifier les nuances ou les enrichir en reflets.

Parmi les coupleurs pouvant être présents dans la composition tinctoriale conforme à l'invention, on peut notamment citer les méta-phénylènediamines, les méta-aminophénols, les métadiphénols, les coupleurs hétérocycliques, et leurs sels d'addition avec un acide.

Lorsqu'ils sont présents, ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IX) suivante : 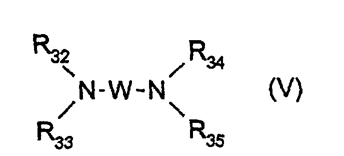 dans laquelle W est un reste propylène éventuellement substitué par un groupement hydroxyle ou un radical alkyle en C₁-C₄ ; R₃₂, R₃₃, R₃₄ et R₃₅, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates percarbonates et persulfates, les peracides, les enzymes telles que les oxydo-réductases à 2 électrons, les peroxydases et les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES DE TEINTURE 1 à 4

On a préparé les compositions tinctoriales conformes à l'invention suivantes (teneurs en grammes) :

| **EXEMPLE** | **1** | **2** | **3** | **4** |
|---|---|---|---|---|
| Diméthylamino-4'-phényl-azo-2-pyridine-N-oxyde (composé de formule (I)) | 0,5 | - | 4,0 | - ' |
| Méthosulfate de diméthylamino-4'-phényl-azo-2-diméthyl-1,3-pyridinium (composé de formule (I)) | - | 0,5 | - | 4,0 |
| Paraphénylènediamine (base d'oxydation) | 0,324 | 0,324 | 0,324 | 0,324 |
| 5-amino 2-méthyl phénol (coupleur) | 0,369 | - | 0,369 | - |
| Méta-aminophénol (coupleur) | - | 0,327 | - | 0,327 |
| Support de teinture commun | (*) | (*) | (*) | (*) |
| Eau déminéralisée qsp | 100 g | 100 g | 100 g | 100 g |

| | | | | |
|---|---|---|---|---|
| (*) : Support de teinture commun : - Alcool oléique polyglycérolé à 2 moles de glycérol 4,0 g - Alcool oléique polyglycérolé à 4 moles de glycérol à 78 % de matières actives (M.A.) 5,69 g M.A. - Acide oléique 3,0 g - Amine oléique à 2 moles d'oxyde d'éthylène vendue sous la dénomination commerciale ETHOMEEN 012 ® par la société AKZO 7,0 g - Laurylamino succinamate de diéthylaminopropyle, sel de sodium à 55 % de M.A. 3,0 g M.A. - Alcool oléique 5,0 g - Diéthanolamide d'acide oléique 12,0 g - Propylèneglycol 3,5 g - Alcool éthylique 16,5 g - Métabisulfite de sodium à en solution aqueuse à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | | | |

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

Les cheveux ont été teints dans les nuances figurant dans le tableau ci-après :

| **EXEMPLE** | **NUANCE OBTENUE** |
|---|---|
| **1** | Rouge intense |
| **2** | Pourpre intense |
| **3** | Rouge intense |
| **4** | Pourpre intense |

## Revendications

1. Composition pour la teinture d'oxydation des fibres kératiniques et en particulier des fibres kératiniques humaines telles que les cheveux, **caractérisée par** le fait qu'elle comprend, dans un milieu approprié pour la teinture :
- au moins une base d'oxydation,
- au moins un colorant direct cationique de formule (I) suivante :
A―N=N―B (I)
dans laquelle :
A représente un groupement choisi parmi les structures A1 à A3 suivantes : 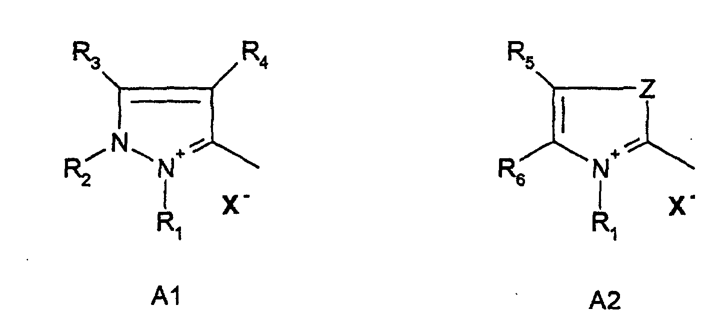 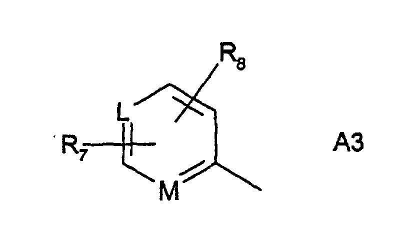 structures A1 à A3 dans lesquelles :
- R₁ désigne un radical alkyle en C₁-C₄, un radical phényle ou un radical phényle substitué par un radical alkyle en C₁-C₄ ou par un atome d'halogène choisi parmi le chlore, le brome, l'iode et le fluor ;
- R₂ désigne un radical alkyle en C₁-C₄ ou un radical phényle ;
- R₃ et R₄, identiques ou différents, représentent un radical alkyle en C₁-C₄, un radical phényle, ou forment ensemble un cycle benzénique substitué par un ou plusieurs radicaux alkyle en C₁-C₄, alcoxy en C₁-C₄ ou nitro ; R₃ peut également désigner un atome d'hydrogène ;
- R₅ et R₆, identiques ou différents, représentent un radical alkyle en C₁-C₄, un radical phényle, ou forment ensemble un cycle benzénique non substitué ou substitué par un ou plusieurs radicaux alkyle en C₁-C₄, alcoxy en C₁-C₄ ou nitro ; R₅ peut également désigner un atome d'hydrogène ;
- R₇ et R₈, identiques ou différents, représentent un atome d'hydrogène ou d'halogène choisi parmi le chlore, le brome, l'iode et le fluor, un radical alkyle en C₁-C₄, alcoxy en C₁-C₄ ou un radical nitro ;
- Z désigne un atome d'oxygène ou de soufre ou un groupement NR₂ dans lequel R₂ peut prendre la même signification que celle indiquée ci-dessus ;
- le somment L représente un groupement -CH, -CR ou -N⁺R₉(X⁻)ᵣ;
- le sommet M représente un groupement -CH, -CR ou.-N⁺R₉(X⁻)ᵣ;
- r est un nombre entier égal à 0 ou 1 ;
- R représente un radical alkyle en C₁-C₄;
- R₉ représente un atome O⁻, un radical alkyle en C₁-C₄ ou alcoxy en C₁-C₄;
- X⁻ représente un anion ;
étant entendu que :
- dans les groupements de formule A3, au moins un des sommets L et M représente un groupement -N⁺R₉(X⁻)ᵣ;
- lorsque R₆ représente un radical alkyle en C₁-C₄ et que Z représente un atome de soufre, alors R₅ est différent d'un radical alkyle en C₁-C₄;
- lorsque R₆ représente un radical alkyle en C₁-C₄ et que Z représente un atome de soufre, et que l'un des radicaux R₁₀ et R₁₁ représente un atome d'hydrogène, alors R₅ est différent d'un atome d'hydrogène ;
- lorsque R₉ désigne O⁻, alors r = 0;
- lorsque L ou M désigne radical un -N⁺R₉(X⁻)ᵣ dans lequel R₉ représente un radical alkyle en C₁-C₄ et r = 1, alors au moins un des radicaux R₇ et R₈ est différent d'un atome d'hydrogène ;
- lorsque L désigne un radical -N⁺R₉(X⁻)ᵣ, alors M représente un groupement -CH ou CR ;
- lorsque M désigne un radical -NR₉(X⁻)ᵣ, alors L représente un groupement -CH ou CR ;
- lorsque Z représente un groupement NR₂ dans lequel R₂ représente un radical alkyle en C₁-C₄, alors au moins un des radicaux R₁, R₅ et R₆ est différent d'un radical alkyle en C₁-C₄;
- lorsque Z représente un atome de soufre et que-R₁ représente un radical alkyle en C₁-C₄, et que l'un des radicaux R₁₀ et R₁₁ représente un atome d'hydrogène, alors R₅ et R₆ ne peuvent pas former ensemble un cycle benzénique non substitué ;
B représente :
**soit (a)** un groupement de structure B1 suivante : 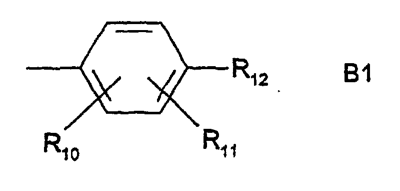 structure B1 dans laquelle,
- R₁₀ représente un atome d'hydrogène, un atome d'halogène choisi parmi le chlore, le brome, l'iode et le fluor, un radical alkyle en C₁-C₄, alcoxy en C₁-C₄, hydroxyle, un groupement -NHR₁₃, -NR₁₄R₁₅, -NHCOalkyle en C₁-C₄, nitro, ou forme avec R₁₁ un cycle à 5 ou 6 chaînons carbonés ou contenant un ou plusieurs hétéroatomes choisis parmi l'azote, l'oxygène ou le soufre ;
- R₁₁ représente un atome d'hydrogène, un atome d'halogène choisi parmi le chlore, le brome, l'iode et le fluor, un radical alkyle en C₁-C₄, alcoxy en C₁-C₄, ou forme avec R₁₂ ou R₁₃ un cycle à 5 ou 6 chaînons carbonés ou contenant un ou plusieurs hétéroatomes choisis parmi l'azote, l'oxygène ou le soufre ;
- R₁₂ représente un atome d'hydrogène, un radical hydroxyle, un radical -NHR₁₃, ou un radical -NR₁₄R₁₅;
- R₁₃ représente un atome d'hydrogène, un radical alkyle en C₁-C₄, monohydroxyalkyle en C₁-C₄, polyhydroxyalkyle en C₂-C₄ ou phényle ;
- R₁₄ et R₁₅, identiques ou différents, représentent un radical alkyle en C₁-C₄, monohydroxyalkyle en C₁-C₄ ou polyhydroxyalkyle en C₂-C₄;
**soit (b)** un groupement hétérocyclique azoté à 5 ou 6 chaînons, un groupement hétérocyclique azoté à 5 ou 6 chaînons renfermant un ou plusieurs hétéroatomes choisi parmi l'oxygène et le soufre et/où un ou plusieurs groupements carbonylés, lesdits hétérocycles pouvant être substitués par un ou plusieurs radicaux alkyle en C₁-C₄, amino ou phényle.

2. Composition selon la revendication 1, **caractérisée par** le fait que l'anion X⁻ est choisi parmi le chlorure, le méthylsulfate, l'éthylsulfate, l'acétate et le perchlorate.

3. Composition selon la revendication 1 ou 2, **caractérisée par** le fait que les groupements hétérocycles azotés à 5 ou 6 chaînons sont choisis parmi les groupements de structure B2 suivante : 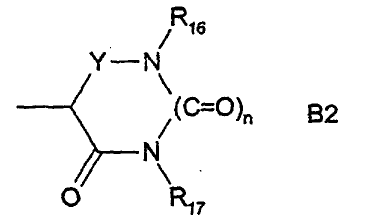 structure B2 dans laquelle :
- R₁₆ et R₁₇, identiques ou différents, représentent un atome d'hydrogène, un radical alkyle en C₁-C₄ ou un radical phényle ;
- Y représente un groupement 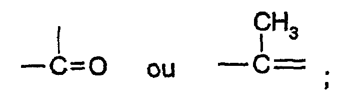
- n est un nombre entier égal à 0 ou 1.

4. Composition selon l'une quelconque des revendications précédentes, **caractérisée par** le fait que la ou les bases d'oxydation sont choisies parmi les paraphénylènediamines, les bases doubles, les para-aminophénols, les ortho aminophénols et les bases d'oxydation hétérocycliques.

5. Composition selon la revendication 4, **caractérisée par** le fait que les paraphénylènediamines sont choisies parmi les composés de formule (II) suivante et leurs sels d'addition avec un acide : 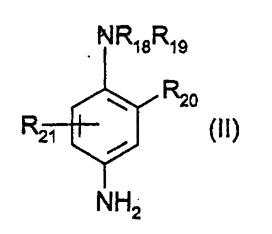 dans laquelle :
- R₁₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₁₉ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₂₀ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₂₁ représente un atome d'hydrogène, d'halogène ou un radical alkyle en C₁-C₄.

6. Composition selon la revendication 5, **caractérisée par** le fait que les paraphénylènediamines de formule (II) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

7. Composition selon la revendication 4, **caractérisée par** le fait que les bases doubles sont choisies parmi les composés de formule (III) suivante, et leurs sels d'addition avec un acide : 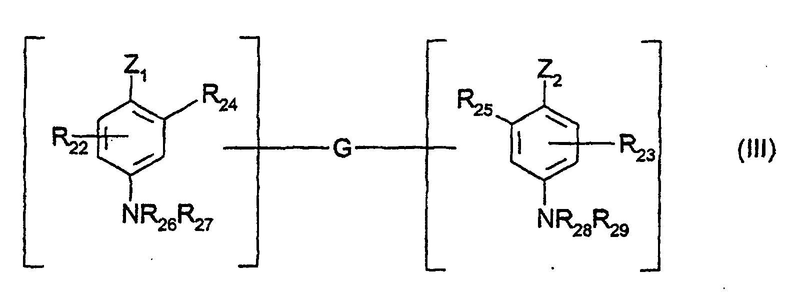 dans laquelle :
- Z₁ et Z₂, identiques ou différents, représentent un radical hydroxyle ou -NH₂ pouvant être substitué par un radical alkyle en C₁-C₄ ou par un bras de liaison G ;
- le bras de liaison G représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₂₂ et R₂₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison G ;
- R₂₄, R₂₅, R₂₆, R₂₇, R₂₈ et R₂₉, identiques ou différents, représentent un atome d'hydrogène, un bras de liaison G ou un radical alkyle en C₁-C₄;
étant entendu que les composés de formule (III) ne comportent qu'un seul bras de liaison G par molécule.

8. Composition selon la revendication 7, **caractérisée par** le fait que les bases doubles de formule (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

9. Composition selon la revendication 4, **caractérisée par** le fait que les para-aminophénols sont choisis parmi les composés de formule (IV) suivante, et leurs sels d'addition avec un acide: 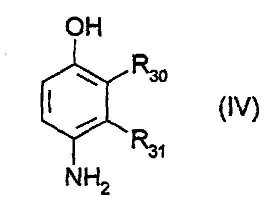 dans laquelle:
- R₃₀ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₃₁ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄), étant entendu qu'au moins un des radicaux R₃₀ et R₃₁ représente un atome d'hydrogène.

10. Composition selon la revendication 9, **caractérisée par** le fait que les para-aminophénols de formule (IV) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

11. Composition selon la revendication 4, **caractérisée par** le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

12. Composition selon la revendication 4, **caractérisée par** le fait que les bases d'oxydation hétérocycliques sont choisies parmi les dérivés pyridiniques, les dérivés pyrimidiniques, les dérivés pyrazoliques, et leurs sels d'addition avec un acide.

13. Composition selon l'une quelconque des revendications précédentes, **caractérisée par** le fait que la ou les bases d'oxydation représentent de 0,0005 à 12 % en poids du poids total de la composition tinctoriale.

14. Composition selon la revendication 13, **caractérisée par** le fait que la ou les bases d'oxydation représentent de 0,005 à 6 % en poids du poids total de la composition tinctoriale.

15. Composition selon l'une quelconque des revendications précédentes, **caractérisée par** le fait que le ou les colorants directs cationiques de formule (I) sont choisis parmi :
- le diméthylamino-4'-phényl-azo-2-pyridine-N-oxyde de formule : 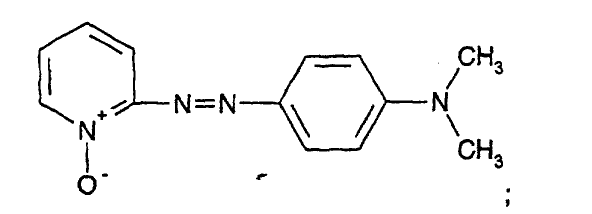
- le diméthylamino-4'-méthyl-2'-phényl-azo-2-pyridine-N-oxyde de formule : 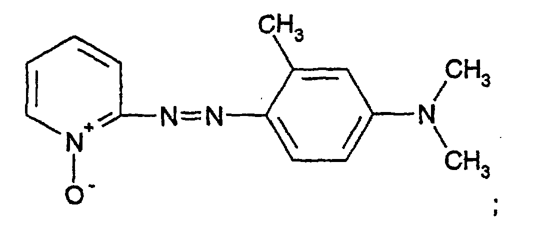
- l'iodure de 2-[(1,3-diamino-6-méthyl-phényl-4)azo]-3-méthyl-benzothiazolium de formule : 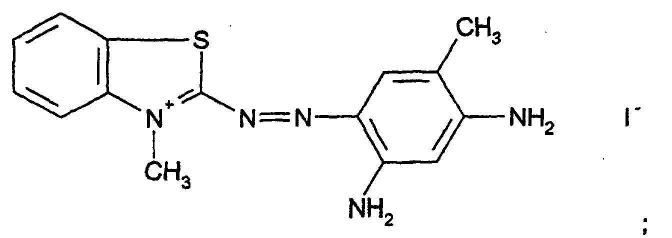
- le chlorure d'hydroxy-6' benzomorpholine-7'-aza-2-méthyl-3-benzothiazolium de formule : 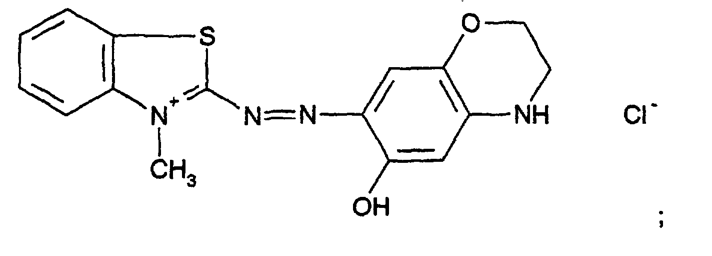
- le perchlorate d'hydroxy-6'-benzomorpholine-7'-aza-2-méthyl-4-phényl-3-thiazolium de formule : 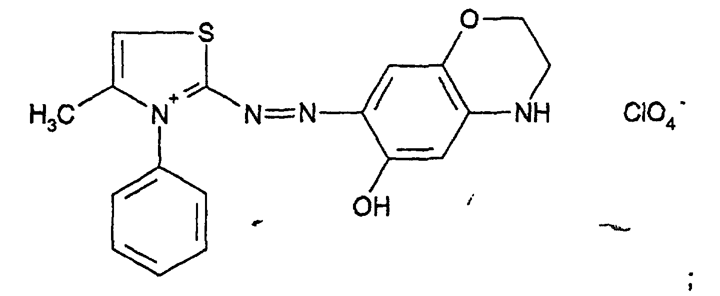
- le perchlorate d'hydroxy-6'-benzomorpholine-7'-azo-2-diphényl-3,4-thiazolium de formule : 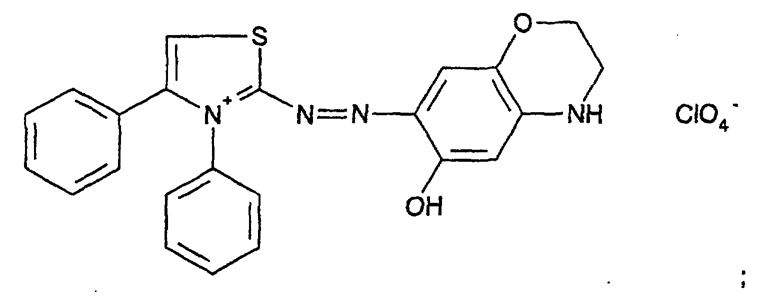
- le chlorure d'hydroxy-6'-benzomorpholine-7'-aza-2-méthyl-3-benzolthiazolium de formule : 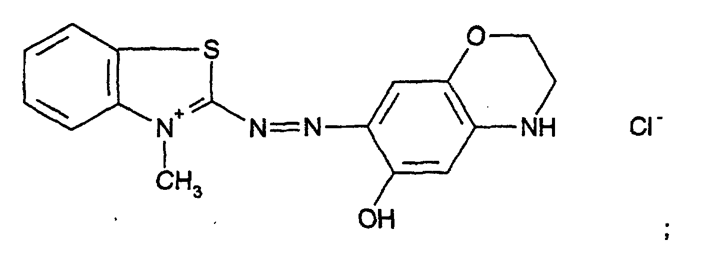
- le perchlorate d'hydroxy-6'-benzomorpholine-7'-aza-2-méthyl-4-phényl-3-thiazolium de formule : 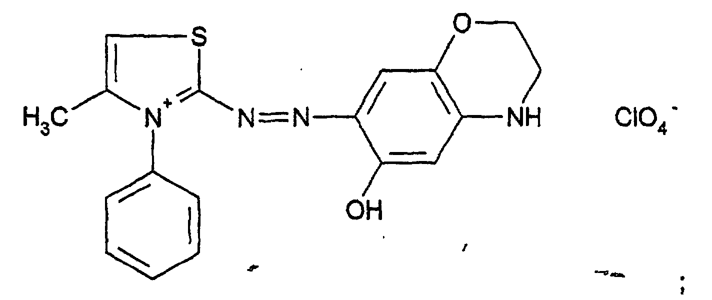
- le diamino-2',4'-méthoxy-5'-phényl-azo-2-pyridine-N-oxyde de formule : 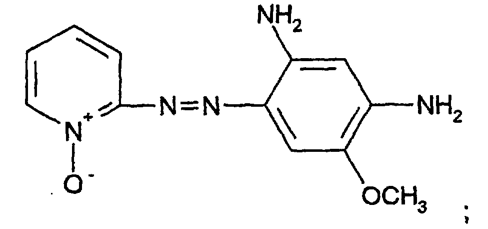
- l'acétamido-2'-hydroxy-4'-méthyl-5'-phényl-azo-2-pyridine N-oxyde de formule : 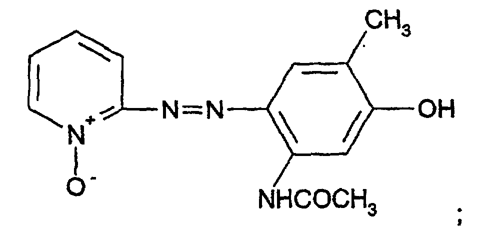
- l'acétamido-2'-diméthylamino-4'-phényl-azo-2-pyridine-N-oxyde de formule : 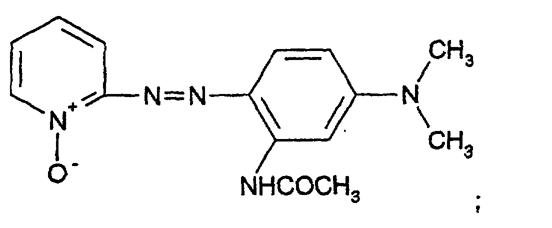
- l'amino-2'-diméthylamino-4'-phényl-azo-2-pyridine-N-oxyde de formule : 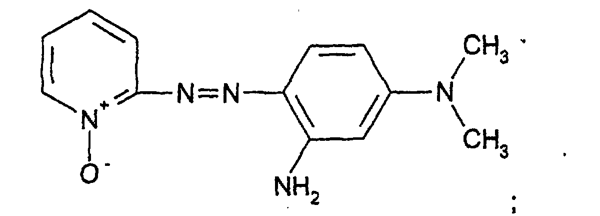
- le perchlorate de diamino-2',4'-méthyl-5'-phényl-azo-2-méthoxy-1-pyridinium de formule : 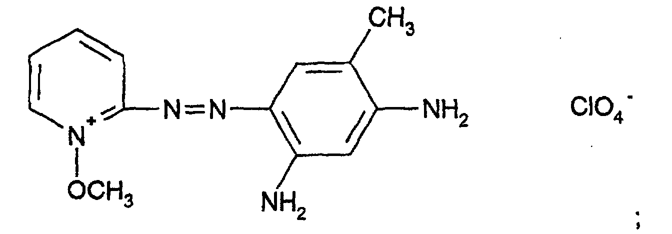
- le diméthyl-2',5'-hydroxy-4'-phényl-azo-2-pyridine-N-oxyde de formule : 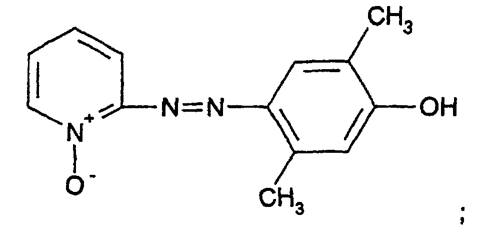
- le diméthylamino-2'-hydroxy-4'-phényl-azo-2-pyridine-N-oxyde de formule : 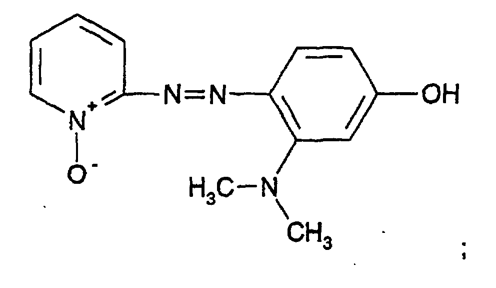
- le diméthylamino-4'-hydroxy-2'-phényl-azo-2-pyridine-N-oxyde de formule : 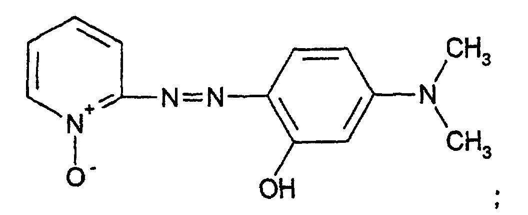
- le diméthyl-3',5'-hydroxy-4'-phényl-azo-2-pyridine-N-oxyde de formule : 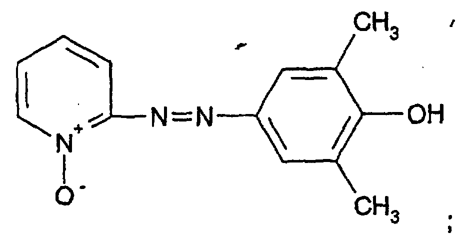
- le diméthylamino-4'-nitro-2'-phényl-azo-2-pyridine-N-oxyde de formule : 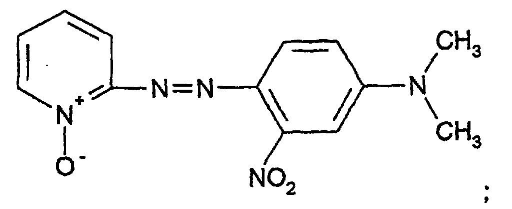
- l'hydroxy-8'-quinoléine-azo-5': 2'-pyridine-N-oxyde de formule : 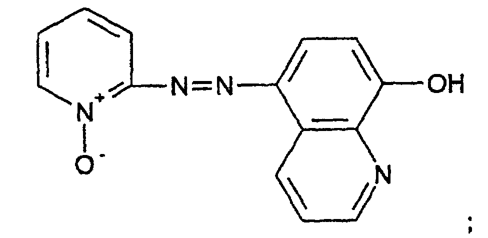
- le méthosulfate de nitro-2'-diméthylamino-4'-phényl-azo-2-méthoxy-1-pyridinium de formule : 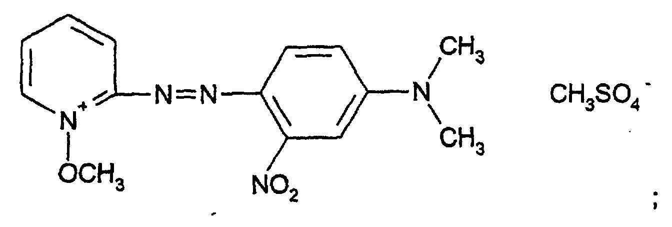
- le diméthylamino-4'-phényl-azo-2-nitro-5-pyridine-N-oxyde de formule : 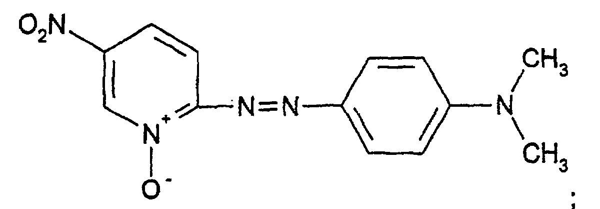
- le diméthylamino-4'-phényl-azo-2-méthyl-6-pyridine-N-oxyde de formule : 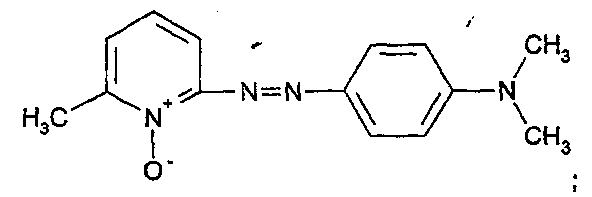
- le diméthylamino-4'-phényl-azo-2-méthyl-5-pyridine-N-oxyde de formule : 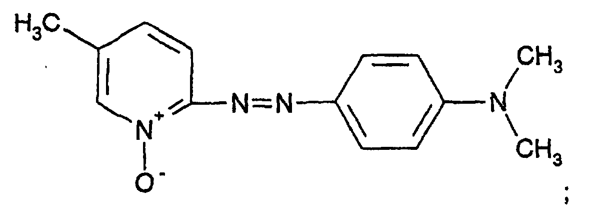
- le diméthylamino-4'-phényl-azo-2-méthyl-3-pyridine-N-oxyde de formule : 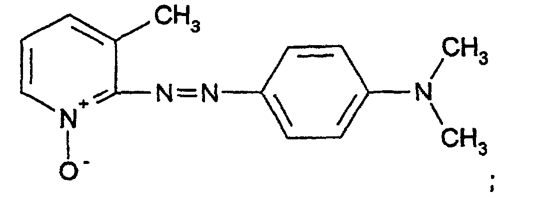
- le diéthylamino-4'-phényl-azo-2-méthyl-3-pyridine-N-oxyde de formule : 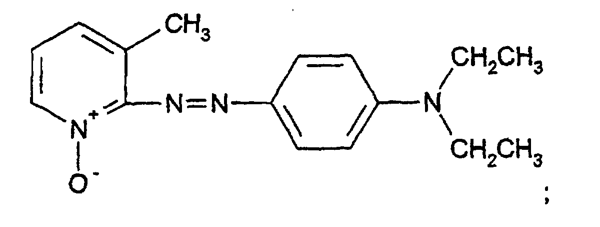
- le diméthylamino-4'-acétylamino-2'-phényl-azo-2-méthyl-3-pyridine-N-oxyde de formule : 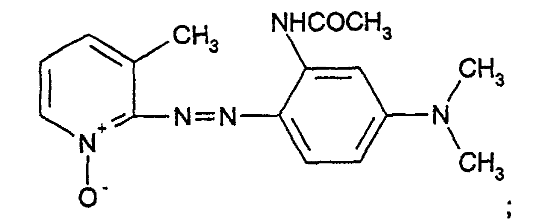
- l'amino-4'-naphtalène-azo-1': 2-méthyl-6-pyridine-N-oxyde de formule : 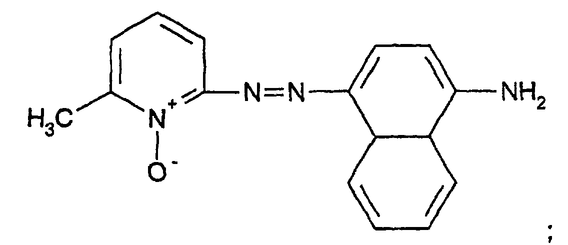
- le di-(β-hydroxyéthyl)amino-4'-phényl-azo-2-méthyl-6-pyridine-N-oxyde de formule : 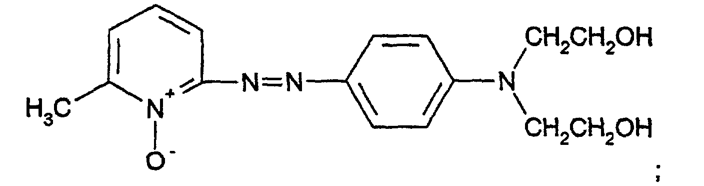
- le diéthylamino-4'-phényl-azo-2-méthyl-6-pyridine-N-oxyde de formule : 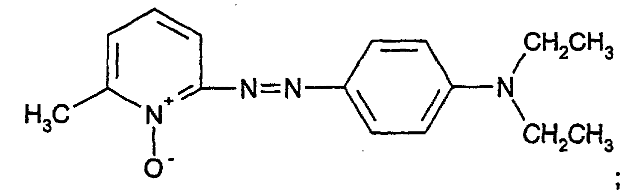
- le diméthyl-2',5'-hydroxy-4'-phényl-azo-2-méthyl-6-pyridine-N-oxyde de formule : 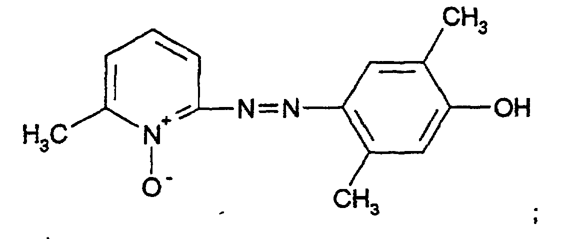
- le di-(β-hydroxyéthyl)amino-4'-phényl-azo-2-méthyl-4-pyridine-N-oxyde de formule : 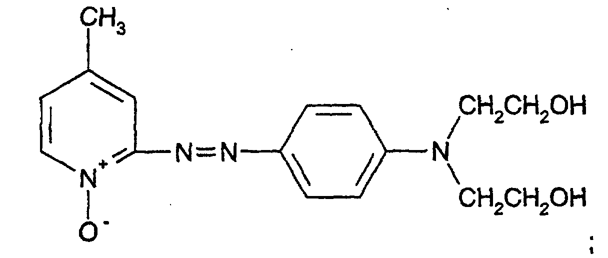
- le diéthylamino-4'-phényl-azo-2-méthyl-4-pyridine-N-oxyde de formule : 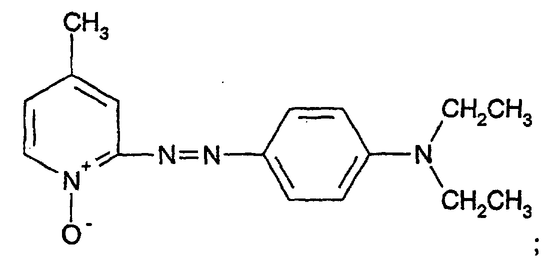
- l'amino-4'-naphtalène-azo-1' : 2-méthyl-4-pyridine-N-oxyde de formule : 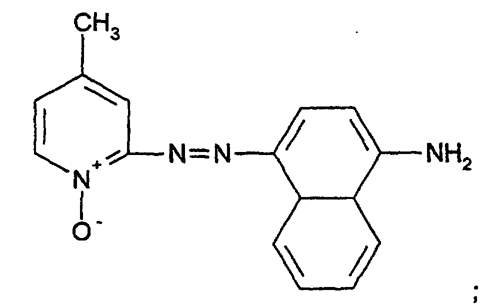
- le diamino-2',4'-méthyl-5'-phényl-azo-2-méthyl-4-pyridine-N-oxyde de formule : 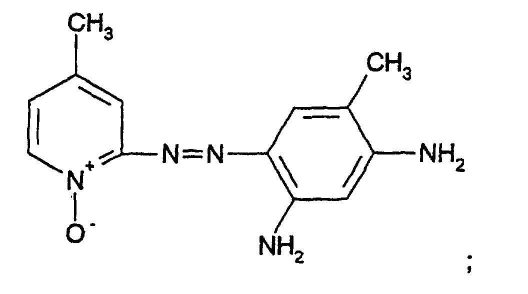
- le diméthylamino-4'-phényl-azo-2-méthyl-4-pyridine-N-oxyde de formule : 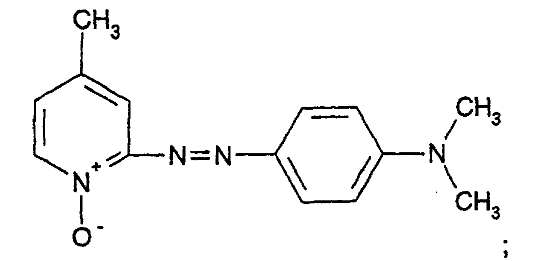
- le diméthylamino-4'-phényl-azo-2-diméthyl-4,6-pyridine-N-oxyde de formule : 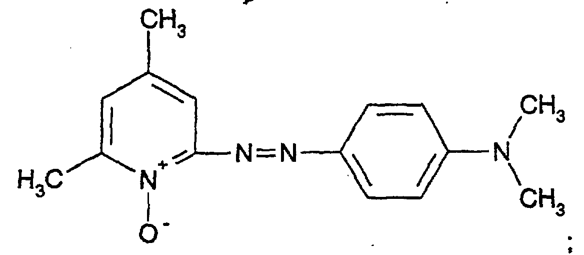
- le diméthyl-2',5'-hydroxy-4'-phényl-azo-2-méthyl-4-pyridine-N-oxyde de formule : 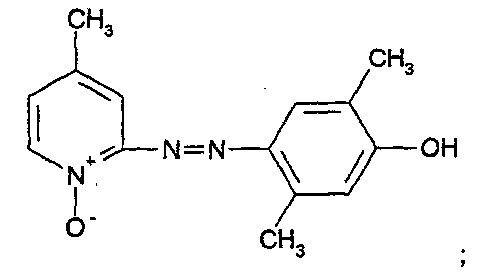
- le diméthylamino-4'-phényl-azo-2-chloro-5-pyridine-N-oxyde de formule : 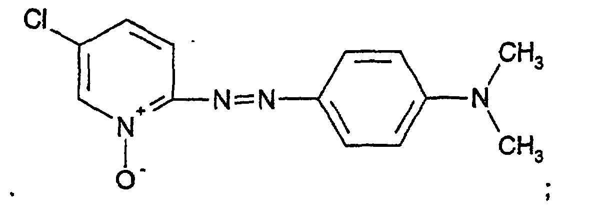
- le diméthylamino-4'-méthyl-2'-phényl-azo-2-chloro-5-pyridine-N-oxyde de formule : 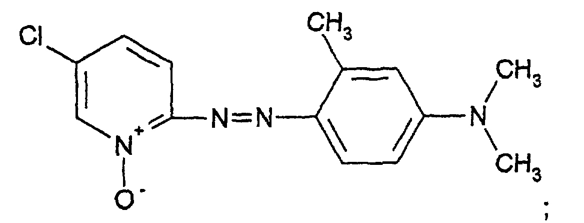
- le diéthylamino-4'-phényl-azo-2-chlorb-5-pyridine-N-oxyde de formule : 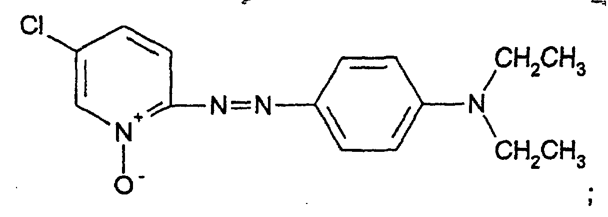
- l'hydroxy-6'-benzomorpholine-azo-7' : 2-pyridine-N-oxyde de formule : 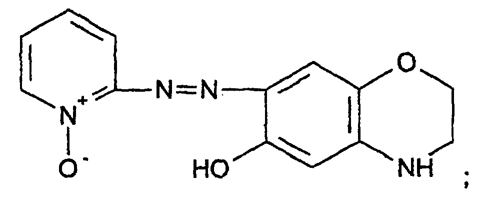
- le méthosulfate d'hydroxy-6'-benzomorpholine-azo-7' : 2-méthoxy-1-pyridinium de formule : 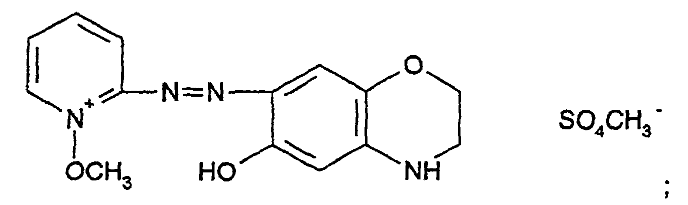
- le diamino-2',4'-méthyl-5'-phényl-azo-2-pyridine-N-oxyde de formule : 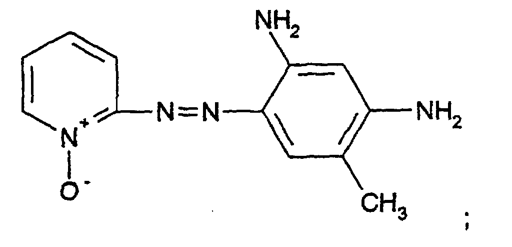
- le méthosulfate de méthyl-2'-oxo-5'-phényl-4'-dihydropyrazolyl-azo-2-méthoxy-1-pyridinium de formule: 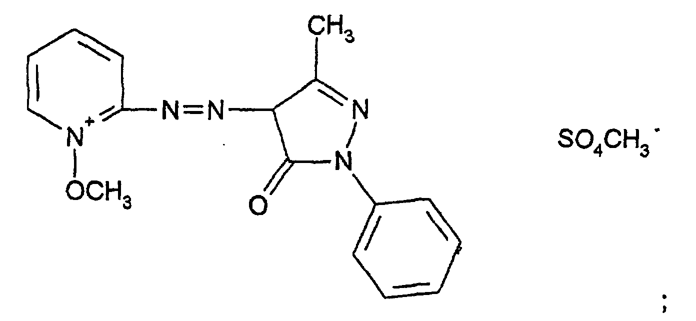
- le méthyl-3'-trioxo-2',4',6'-hexahydro-pyrimidinyl-azo-2-pyridine-N-oxyde de formule : 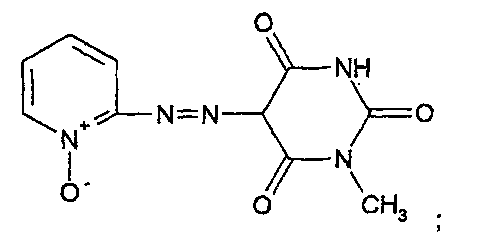
- l'amino-4'-phényl-azo-2-pyridine-N-oxyde de formule : 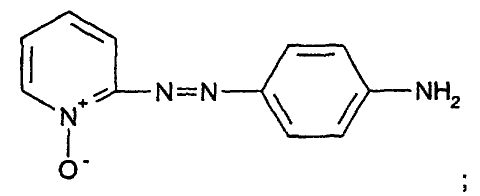
- le di-(β-hydroxyéthyl)amino-4'-phényl-azo-2-pyridine-N-oxyde de formule : 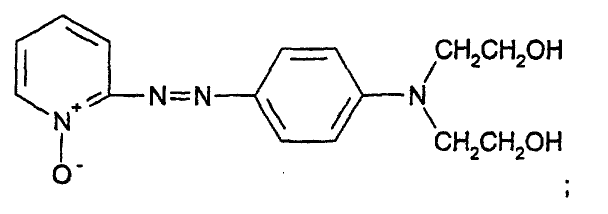
- le (N-phénylamino)-4'-phényl-azo-2-pyridine-N-oxyde de formule : 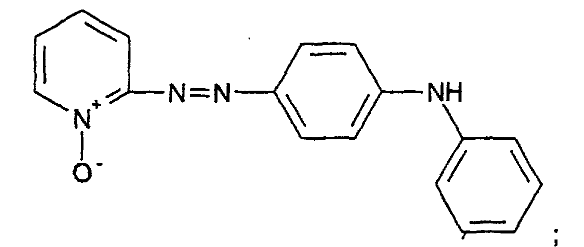
- le (N-phénylamino)-4'-phényl-azo-2-méthyl-3-pyridine-N-oxyde de formule : 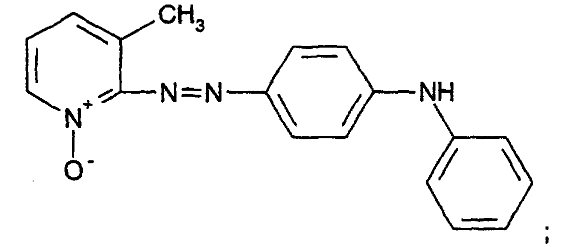
- le méthosulfate d'amino-4'-phényl-azo-2-diméthyl-1,3-pyridinium de formule : 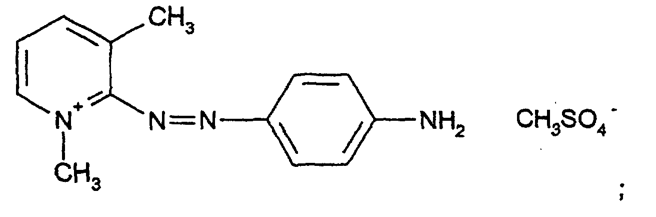
- le méthosulfate de di-(β-hydroxyéthyl)amino-4'-phényl-azo-2-diméthyl-1,4-pyridinium de formule : 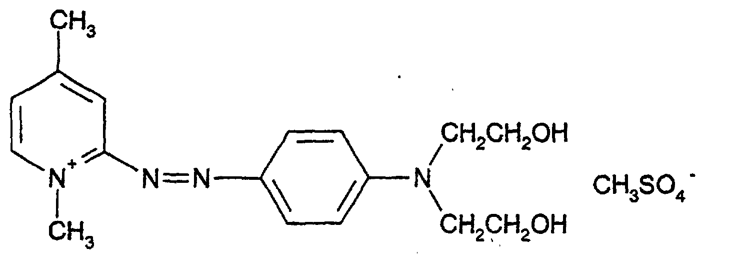
- le méthosulfate de diméthylamino-4'-phényl-azo-2-diméthyl-1,3-pyridinium de formule : 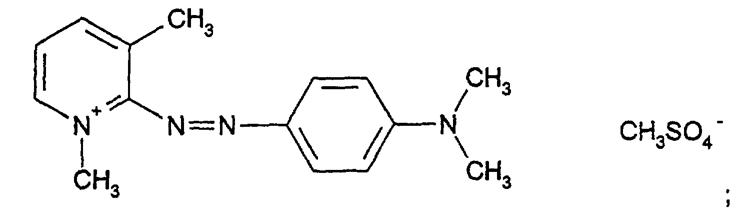
- le méthosulfate de N-phénylamino-4'-phényl-azo-2-diméthyl-1,6-pyridinium de formule : 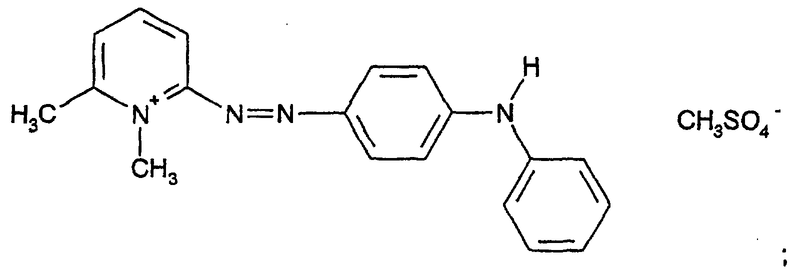
- le méthosulfate de diméthylamino-4'-phényl-azo-2-diméthyl-1,5-pyridinium de formule : 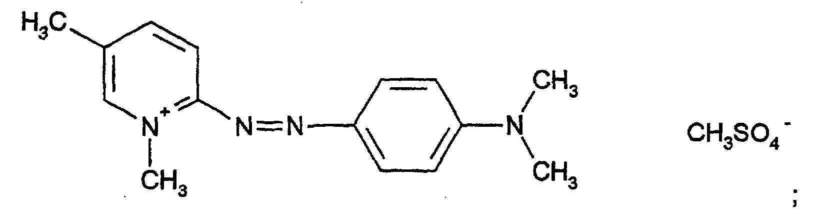
- le méthosulfate de diméthylamino-4'-phényl-azo-2-diméthyl-1,6-pyridinium de formule: 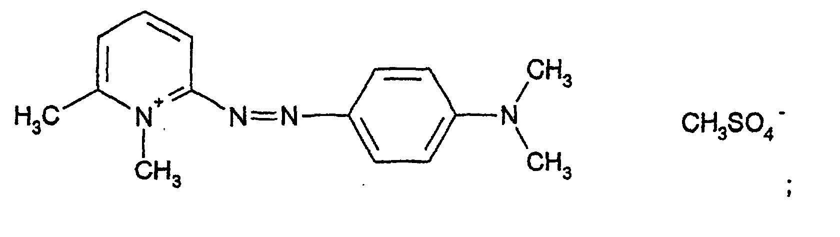
- le trioxo-2',4',6'-trioxo-hexahydro-pyrimidinyl-azo-2-pyridine-N-oxyde de formule : 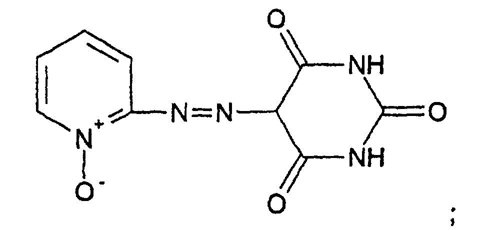
- le perchlorate de diméthylamino-4'-phényl-azo-2-diméthyl-1,3-benzimidazolium de formule : 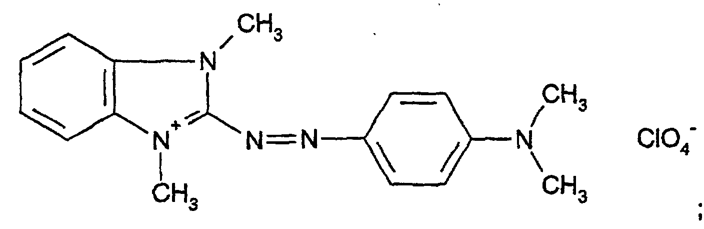 et
- le diamino-2',4'-pyridine-3'-azo-2-pyridine-N-oxyde de formule :

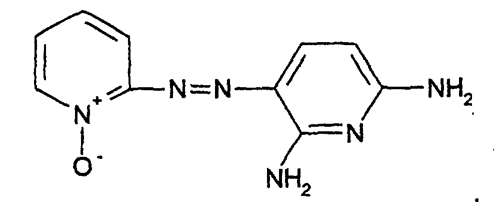

16. Composition selon l'une quelconque des revendications précédentes, **caractérisée par** le fait que le ou les colorants directs cationiques de formule (I) représentent de 0,001 à 10 % en poids du poids total de la composition tinctoriale.

17. Composition selon la revendication 16, **caractérisée par** le fait que le ou les colorants directs cationiques de formule (I) représentent de 0,01 à 5 % en poids du poids total de la composition tinctoriale.

18. Composition selon l'une quelconque des revendications précédentes, **caractérisée par** le fait qu'elle contient un ou plusieurs coupleurs et/ou un ou plusieurs colorants directs additionnels différents des colorants directs cationiques de formule (I) telle que définie à la revendication 1.

19. Composition selon la revendication 18, **caractérisée par** le fait que le ou les coupleurs sont choisis parmi les méta-phénylènediamines, les métaaminophénols, les métadiphénols, les coupleurs hétérocycliques, et leurs sels d'addition avec un acide.

20. Composition selon la revendication 18 ou 19, **caractérisée par** le fait que le ou les coupleurs représentent de 0,0001 à 10 % en poids du poids total de la composition tinctoriale.

21. Composition selon la revendication 20, **caractérisée par** le fait que le ou les coupleurs représentent de 0,005 à 5 % en poids du poids total de la composition tinctoriale.

22. Composition selon l'une quelconque des revendications précédentes, **caractérisée par** le fait que les sels d'addition avec un acide sont choisis parmi les chlorhydrates, les bromhydrates, les sulfates et les tartrates, les lactates et les acétates.

23. Procédé de teinture des fibres kératiniques et en particulier des fibres kératiniques humaines telles que les cheveux, **caractérisé par** le fait qu'on applique sur lesdites fibres au moins une composition tinctoriale telle que définie à l'une quelconque des revendications 1 à 22, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

24. Procédé selon la revendication 23, **caractérisé par** le fait que l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

25. Dispositif à plusieurs compartiments, ou "kit" de teinture à plusieurs compartiments, dont un premier compartiment renferme une composition tinctoriale telle que définie à l'une quelconque des revendications 1 à 22 et un second compartiment renferme une composition oxydante.

## Patentansprüche

1. Zusammensetzung zum oxidativen Färben von Keratinfasern und insbesondere menschlichen Keratinfasern, wie dem Haar, **dadurch gekennzeichnet, daß** sie in einem zum Färben geeigneten Medium enthält:
- mindestens eine Oxidationsbase, und
- mindestens einen kationischen Direktfarbstoff der folgenden Formel (I):
A - N = N - B (I),
worin:
A eine Gruppe bedeutet, die unter den folgenden Strukturen A1 bis A3 ausgewählt ist: 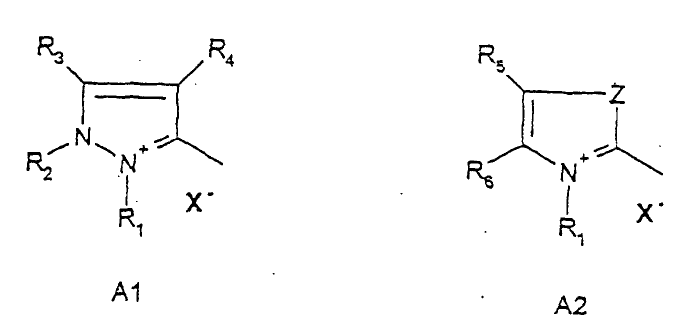 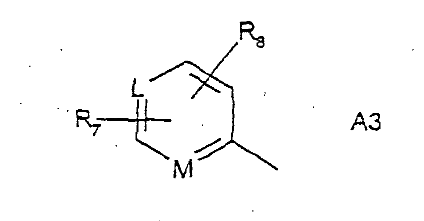 wobei in den Strukturen A1 bis A3 bedeuten:
- R₁ eine C₁₋₄-Alkylgruppe, eine Phenylgruppe oder eine Phenylgruppe, die mit C₁₋₄-Alkyl oder einem Halogenatom, das unter Chlor, Brom, Iod und Fluor ausgewählt ist, substituiert ist;
- R₂ eine C₁₋₄-Alkylgruppe oder eine Phenylgruppe;
- R₃ und R₄, die identisch oder voneinander verschieden sind, eine C₁₋₄-Alkylgruppe oder eine Phenylgruppe oder sie bilden gemeinsam einen Benzolring, der mit einer oder mehreren Gruppen C₁₋₄-Alkyl, C₁₋₄-Alkoxy oder Nitro substituiert ist; R₃ kann auch ein Wasserstoffatom bedeuten;
- R₅ und R₆, die identisch oder voneinander verschieden sind, eine C₁₋₄-Alkylgruppe oder eine Phenylgruppe oder sie bilden gemeinsam einen Benzolring, der unsubstituiert vorliegt oder mit einer oder mehreren Gruppen C₁₋₄-Alkyl, C₁₋₄-Alkoxy oder Nitro substituiert ist; R₅ kann auch ein Wasserstoffatom bedeuten;
- R₇ und R₈, die identisch oder voneinander verschieden sind, ein Wasserstoffatom oder ein Halogenatom, das unter Chlor, Brom, Iod und Fluor ausgewählt ist, C₁₋₄-Alkyl, C₁₋₄-Alkoxy oder Nitro;
- Z ein Sauerstoffatom, ein Schwefelatom oder eine Gruppe NR₂, worin R₂ die oben angegebenen Bedeutungen aufweisen kann;
- L eine Gruppe -CH, -CR oder -N⁺R₉(X⁻)ᵣ;
- M eine Gruppe -CH, -CR oder -N⁺R₉(X⁻)ᵣ;
- r 0 oder 1;
- R C₁₋₄-Alkyl;
- R₉ ein Atom O-, C₁₋₄-Alkyl oder C₁₋₄-Alkoxy;
- X- ein Anion;
mit der Maßgabe, daß
- in den Gruppen der Formel A3 mindestens eine der Gruppen L und M eine Gruppe -N⁺R₉(X⁻)ᵣ bedeutet;
- wenn R₆ C₁₋₄-Alkyl bedeutet und Z ein Schwefelatom ist, R₅ von C₁₋₄-Alkyl verschieden ist;
- wenn R₆ C₁₋₄-Alkyl bedeutet, Z ein Schwefelatom ist und eine der Gruppen R₁₀ und R₁₁ Wasserstoff bedeutet, R₅ von Wasserstoff verschieden ist;
- wenn R₉ O⁻ bedeutet, r = 0;
- wenn L oder M eine Gruppe -N⁺R₉(X⁻)ᵣ bedeuten, worin R₉ eine C₁₋₄-Alkylgruppe ist und r = 1, mindestens eine der Gruppen R₇ und R₈ von Wasserstoff verschieden ist;
- wenn L eine Gruppe -N⁺R₉(X⁻)ᵣ bedeutet, M eine Gruppe -CH oder -CR ist;
- wenn M eine Gruppe -N⁺R₉(X⁻)ᵣ bedeutet, L eine Gruppe -CH oder -CR ist;
- wenn Z eine Gruppe NR₂ ist, worin R₂ eine C₁₋₄-Alkylgruppe ist, mindestens eine der Gruppen R₁, R₅ und R₆ von C₁₋₄-Alkyl verschieden ist;
- wenn Z ein Schwefelatom bedeutet, R₁ eine C₁₋₄-Alkylgruppe ist und eine der beiden Gruppen R₁₀ und R₁₁ Wasserstoff bedeutet, die Gruppen R₅ und R₆ keinen unsubstituierten Benzolring bilden können;
B bedeutet:
entweder (a) eine Gruppe der folgenden Struktur B1: 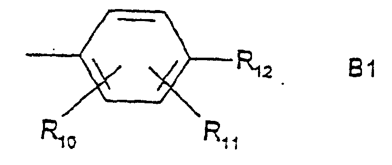 wobei in der Struktur B1 bedeuten:
- R₁₀ ein Wasserstoffatom, ein Halogenatom, das unter Chlor, Brom, Iod und Fluor ausgewählt ist, C₁₋₄-Alkyl, C₁₋₄-Alkoxy, Hydroxy, -NHR₁₃, -NR₁₄R₁₅, -NHCO-C₁₋₄-alkyl oder Nitro oder R₁₀ bildet mit R₁₁ einen 5- oder 6-gliedrigen Ring, der aus Kohlenstoffatomen besteht oder ein oder mehrere Heteroatome enthält, die unter Stickstoff, Sauerstoff oder Schwefel ausgewählt sind; R₁₁ ein Wasserstoffatom, ein Halogenatom, das unter Chlor, Brom, Iod und Fluor ausgewählt ist, C₁₋₄-Alkyl oder C₁₋₄-Alkoxy oder R₁₁ bildet mit R₁₂ oder R₁₃ einen 5- oder 6-gliedrigen Ring, der aus Kohlenstoffatomen besteht oder ein oder mehrere Heteroatome enthält, die unter Stickstoff, Sauerstoff oder Schwefel ausgewählt sind;
- R₁₂ Wasserstoff, Hydroxy, -NHR₁₃ oder-NR₁₄R₁₅;
- R₁₃ Wasserstoff, C₁₋₄-Alkyl, C₁₋₄-Monohydroxyalkyl, C₂₋₄-Polyhydroxyalkyl oder Phenyl;
- R₁₄ und R₁₅, die identisch oder voneinander verschieden sind, C₁₋₄-Alkyl, C₁₋₄-Monohydroxyalkyl oder C₂₋₄-Polyhydroxyalkyl;
oder (b) eine stickstoffhaltige, 5- oder 6-gliedrige ,heterocyclische Gruppe, eine stickstoffhaltige, 5- oder 6-gliedrige, heterocyclische Gruppe, die ein oder mehrere Heteroatome, die unter Sauerstoff und Schwefel ausgewählt sind, und/oder eine oder mehrere carbonylhaltige Gruppen enthält, wobei die Heterocyclen mit einer oder mehreren C₁₋₄-Alkylgruppen, Aminogruppen oder Phenylgruppen substituiert sein können.

2. Zusammensetzung nach Anspruch 1, **dadurch gekennzeichnet, daß** das Anion X- unter Chlorid, Methylsulfat, Ethylsulfat, Acetat und Perchlorat ausgewählt ist.

3. Zusammensetzung nach Anspruch 1 oder 2, **dadurch gekennzeichnet, daß** die stickstoffhaltigen, 5- oder 6-gliedrigen, heterocyclischen Gruppen unter den Gruppen der folgenden Struktur B2 ausgewählt sind: 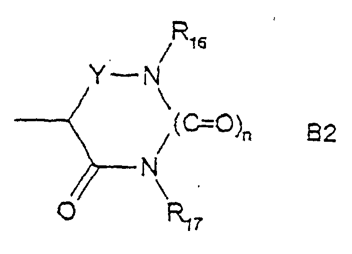 wobei in der Struktur B2 bedeuten:
- R₁₆ und R₁₇, die identisch oder voneinander verschieden sind, Wasserstoff, C₁₋₄-Alkyl oder Phenyl;
- Y eine Gruppe 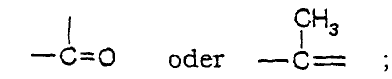 und
- n 0 oder 1.

4. Zusammensetzung nach einem der vorhergehenden Ansprüche, **dadurch gekennzeichnet, daß** die Oxidationsbase(n) unter den p-Phenylendiaminen, Doppelbasen, p-Aminophenolen, o-Aminophenolen und den heterocyclischen Oxidationsbasen ausgewählt sind.

5. Zusammensetzung nach Anspruch 4, **dadurch gekennzeichnet, daß** die p-Phenylendiamine unter den Verbindungen der folgenden Formel (II) und deren Additionssalzen mit einer Säure ausgewählt sind: 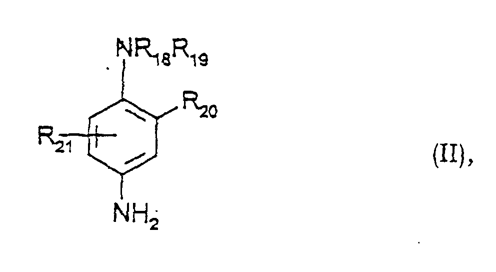 worin bedeuten:
R₁₈ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
R₁₉ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
R₂₀ Wasserstoff, Halogen, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
R₂₁ Wasserstoff, Halogen oder C₁₋₄-Alkyl.

6. Zusammensetzung nach Anspruch 5, **dadurch gekennzeichnet, daß** die p-Phenylendiamine der Formel (II) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-bis(β-Hydroxyethyl)amino-2-methyl-anilin, 4-N,N-bis(β-Hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin und deren Additionssalzen mit einer Säure ausgewählt sind.

7. Zusammensetzung nach Anspruch 4, **dadurch gekennzeichnet, daß** die Doppelbasen unter den Verbindungen der folgenden Formel (III) und deren Additionssalze mit einer Säure ausgewählt sind: 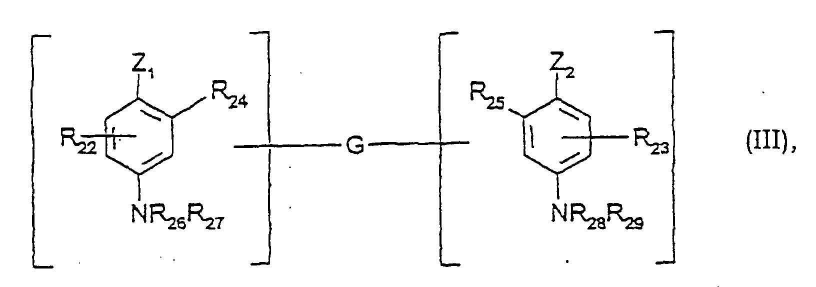 worin bedeuten:
Z₁ und Z₂, die identisch oder voneinander verschieden sind, Hydroxy oder eine NH₂-Gruppe, die mit C₁₋₄-Alkyl oder einer Verbindungsgruppe G substituiert sein kann,
die Verbindungsgruppe G eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden und gegebenenfalls mit einer oder mehreren Hydroxy- oder C₁₋₆-Alkoxygruppen substituiert sein kann,
R₂₂ und R₂₃ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe G,
R₂₄, R₂₅, R₂₆, R₂₇, R₂₈ und R₂₉, die identisch oder voneinander verschieden sind, Wasserstoff, eine Verbindungsgruppe G oder C₁₋₄-Alkyl,
mit der Maßgabe, daß die Verbindungen der Formel (III) nur eine Verbindungsgruppe G pro Molekül aufweisen.

8. Zusammensetzung nach Anspruch 7, **dadurch gekennzeichnet, daß** die Doppelbasen der Formel (III) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis(4'-amino, 3'-methylphenyl)-ethylendiamin, 1,8-Bis(2,5-diaminophenoxy)-3,5-dioxaoctan und deren Additionssalzen mit einer Säure ausgewählt sind.

9. Zusammensetzung nach Anspruch 4, **dadurch gekennzeichnet, daß** die p-Aminophenole unter den Verbindungen der folgenden Formel (IV) und deren Additionssalze mit einer Säure ausgewählt sind: 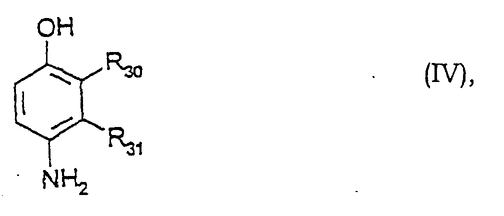 worin bedeuten:
R₃₀ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄-aminoalkyl,
R₃₁ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, daß mindestens eine der Gruppen R₃₀ oder R₃₁ Wasserstoff bedeutet.

10. Zusammensetzung nach Anspruch 9, **dadurch gekennzeichnet, daß** die p-Aminophenole der Formel (IV) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

11. Zusammensetzung nach Anspruch 4, **dadurch gekennzeichnet, daß** die o-Aminophenole unter 2-Aminophenol, 2-Amino-5-methylphenol, 2-Amino-6-methyl-phenol, 5-Acetamido-2-amino-phenol und deren Additionssalzen mit einer Säure ausgewählt sind.

12. Zusammensetzung nach Anspruch 4, **dadurch gekennzeichnet, daß** die heterocyclischen Oxidationsbasen unter den Pyridinderivaten, Pyrimidinderivaten, Pyrazolderivaten und deren Additionssalzen mit einer Säure ausgewählt sind.

13. Zusammensetzung nach einem der vorhergehenden Ansprüche, **dadurch gekennzeichnet, daß** die Oxidationsbase(n) 0,0005 bis 12 Gew.-% des Gesamtgewichts der Farbmittelzusammensetzung ausmachen.

14. Zusammensetzung nach Anspruch 13, **dadurch gekennzeichnet, daß** die Oxidationsbase(n) 0,005 bis 6 Gew.-% des Gesamtgewichts der Farbmittelzusammensetzung ausmachen.

15. Zusammensetzung nach einem der vorhergehenden Ansprüche, **dadurch gekennzeichnet, daß** der oder die kationischen Direktfarbstoffe der Formel (I) ausgewählt sind unter:
- 2-(4'-Dimethylamino-phenyl-azo)-pyridin-N-oxid der Formel: 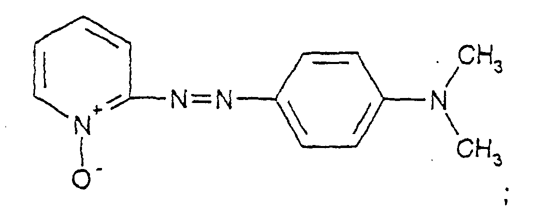
- 2-(4'-Dimethylamino-2'-methyl-phenyl-azo)-pyridin-N-oxid der Formel: 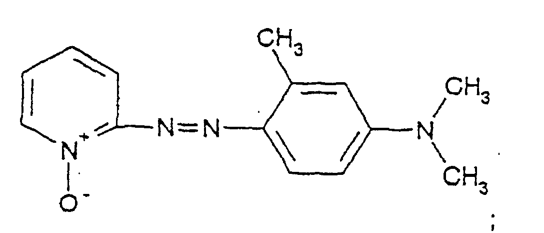
- 2-[(1,3-Diamino-6-methyl-phenyl-4)-azo]-3-methylbenzothiazoliumiodid der Formel: 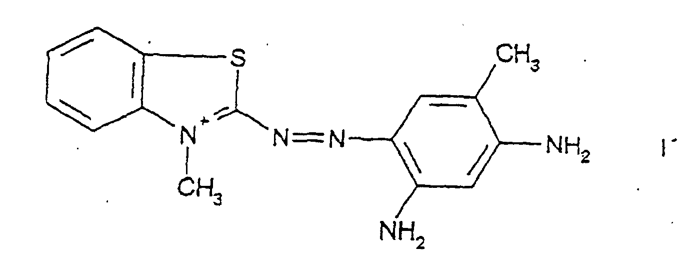
- 2-(6'-Hydroxy-benzomorpholin-7'-azo)-3-methyl-benzothiazoliumchlorid der Formel: 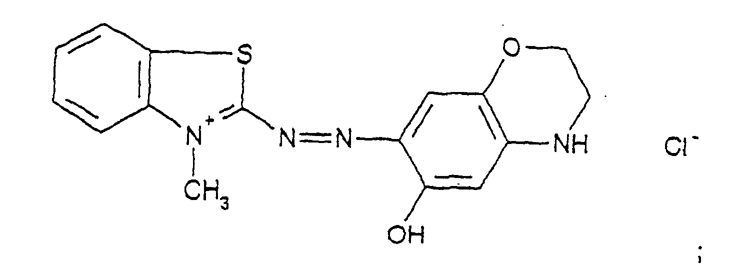
- 2-(6'-Hydroxy-benzomorpholin-7'-azo)-4-methyl-3-phenylthiazoliumperchlorat der Formel: 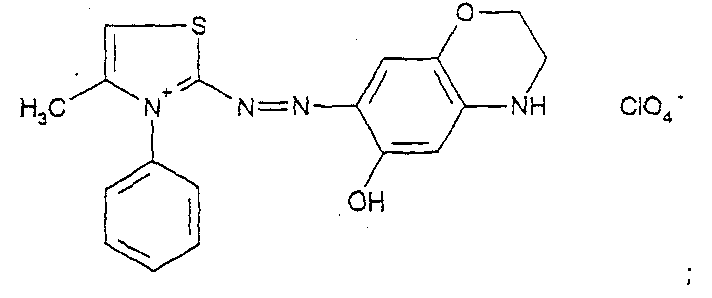
- 2-(6'-Hydroxy-benzomorpholin-7'-azo)-3,4-diphenylthiazoliumperchlorat der Formel: 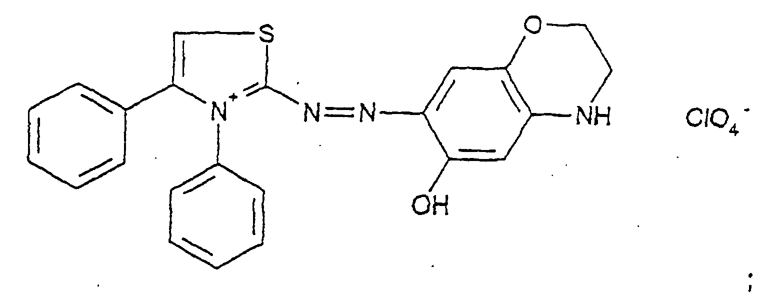
- 2-(6'-Hydroxy-benzomorpholin-7'-azo)-3-methyl-benzothiazoliumchlorid der Formel: 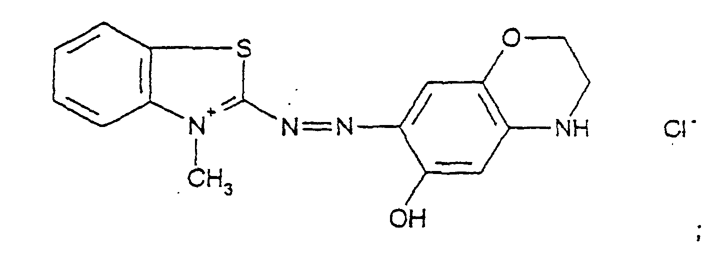
- 2-(6'-Hydroxy-benzomorpholin-7'-azo)-4-methyl-3-phenylthiazoliumperchlorat der Formel: 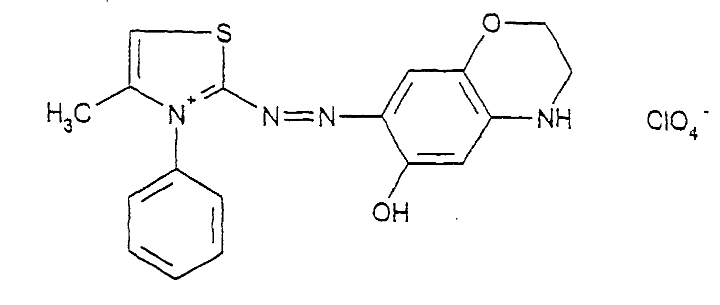
- 2-(2',4'-Diamino-5'-methoxy-phenyl-azo)-pyridin-N-oxid der Formel: 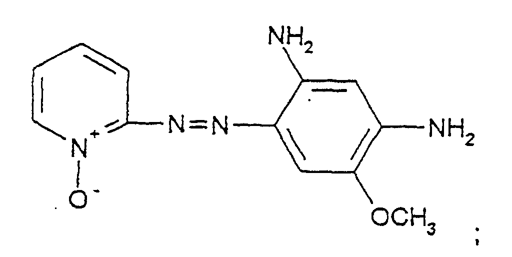
- 2-(2'-Acetamido-4'-hydroxy-5'-methyl-phenyl-azo)-pyridin-N-oxid der Formel: 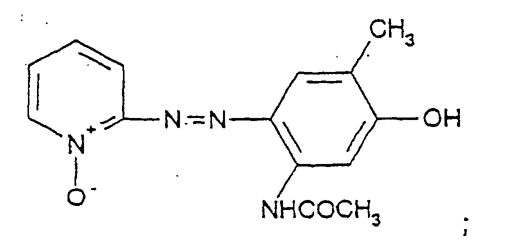
- 2-(2'-Acetamido-4'-dimethylamino-phenyl-azo)-pyridin-N-oxid der Formel: 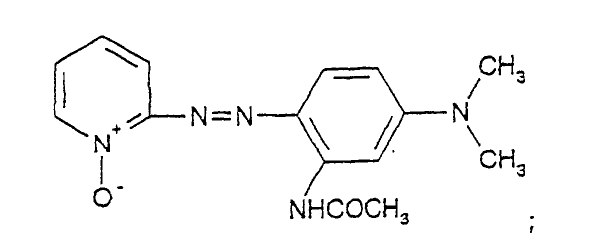
- 2-(2'-Amino-4'-dimethylamino-phenyl-azo)-pyridin-N-oxid der Formel: 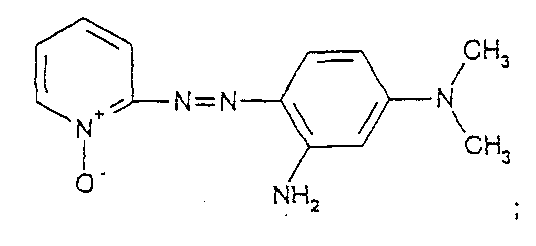
- 2 - (2',4'-Diamino-5'-methyl-phenyl-azo)-1-methoxy-pyridiniumperchlorat der Formel: 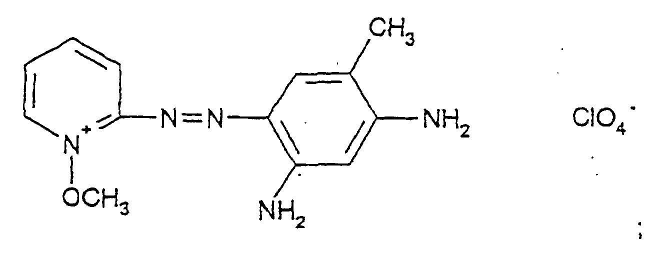
- 2-(2',5'-Dimethyl-4'-hydroxy-phenyl-azo)-pyridin-N-oxid der Formel: 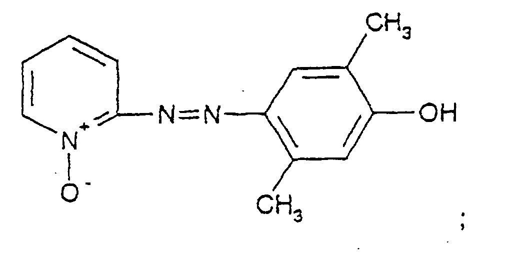
- 2-(2'-Dimethylamino-4'-hydroxy-phenyl-azo)-pyridin-N-oxid der Formel: 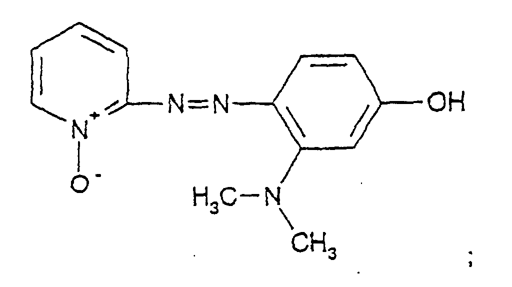
- 2-(4'-Dimethylamino-2'-hydroxy-phenyl-azo)-pyridin-N-oxid der Formel: 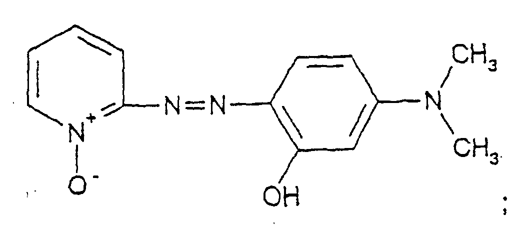
- 2-(3',5'-Dimethyl-4'-hydroxy-phenyl-azo)-pyridin-N-oxid der Formel: 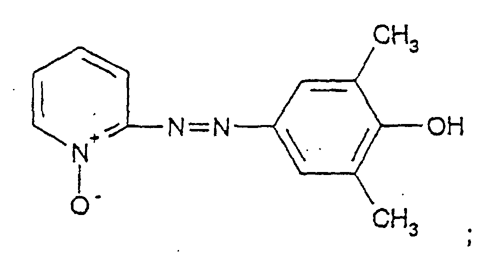
- 2-(4'-Dimethylamino-2'-nitro-phenyl-azo)-pyridin-N-oxid der Formel: 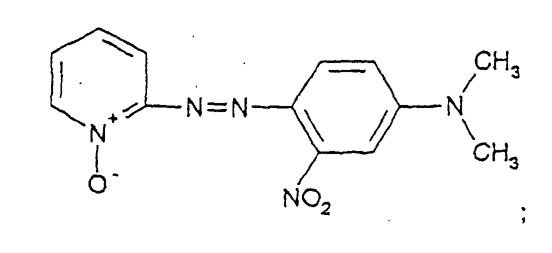
- 2-(8'-Hydroxy-chinolin-5'-azo)-pyridin-N-oxid der Formel: 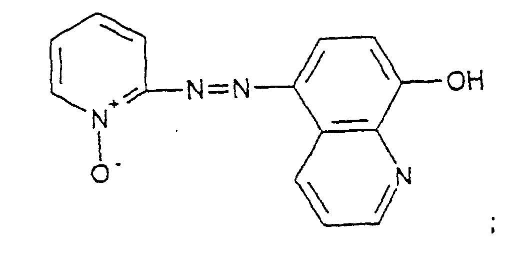
- 2-(2'-Nitro-4'-dimethylamino-phenyl-azo)-1-methoxy-pyridiniummethosulfat der Formel: 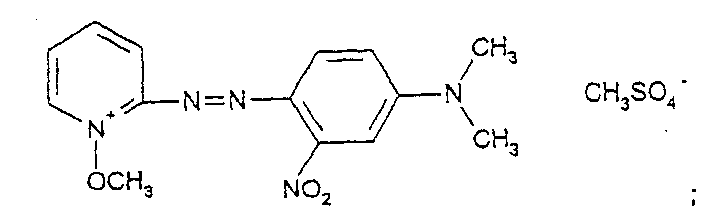
- 2-(4'-Dimethylamino-phenyl-azo)-5-nitro-pyridin-N-oxid der Formel: 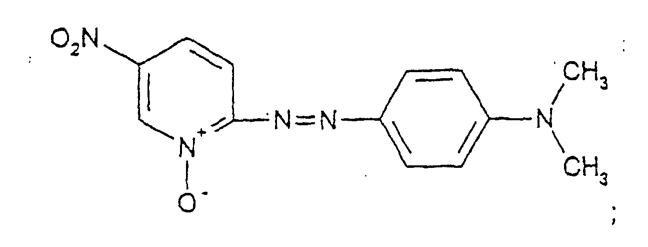
- 2-(4'-Dimethylamino-phenyl-azo)-6-methyl-pyridin-N-oxid der Formel: 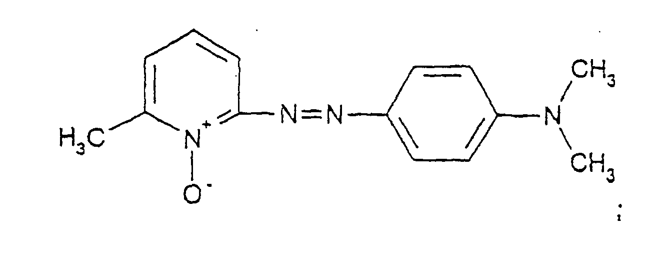
- 2-(4'-Dimethylamino-phenyl-azo)-5-methyl-pyridin-N-oxid der Formel: 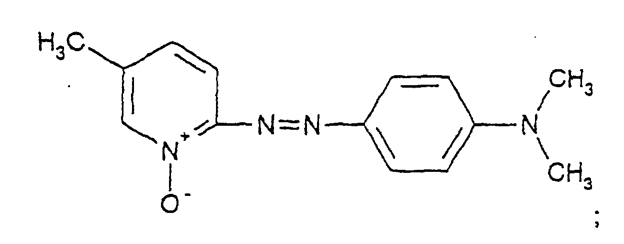
- 2-(4'-Dimethylamino-phenyl-azo)-3-methyl-pyridin-N-oxid der Formel: 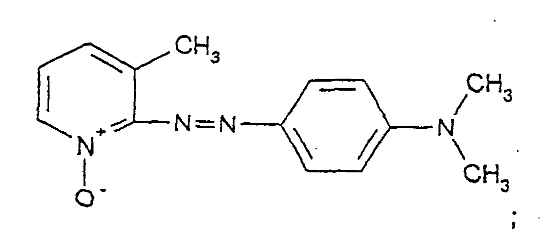
- 2-(4'-Diethylamino-phenyl-azo)-3-methyl-pyridin-N-oxid der Formel: 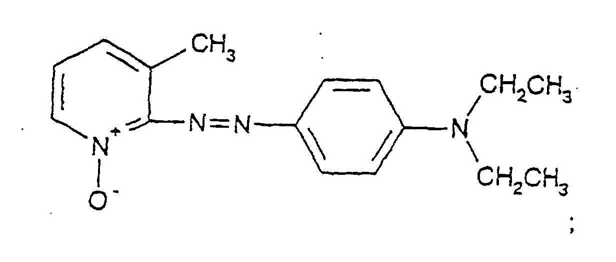
- 2-(4'-Dimethylamino-2'-acetylamino-phenyl-azo)-3-methylpyridin-N-oxid der Formel: 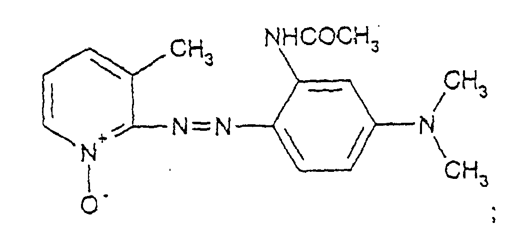
- 2-(4'-Amino-naphthalin-1'-azo)-6-methyl-pyridin-N-oxid der Formel: 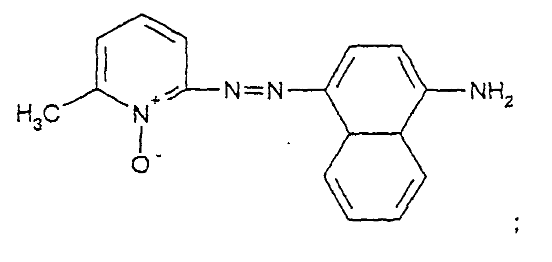
- 2-(4'-Di(β-hydroxyethyl)amino-phenyl-azo)-6-methyl-pyridin-N-oxid der Formel: 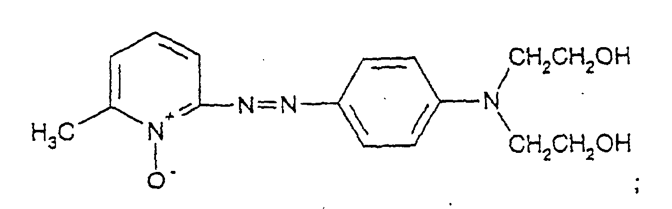
- 2-(4'-Diethylamino-phenyl-azo)-6-methyl-pyridin-N-oxid der Formel: 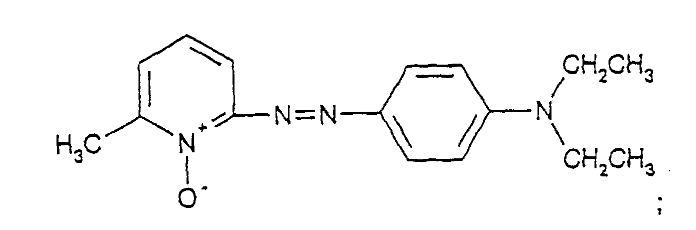
- 2-(2',5'-Dimethyl-4'-hydroxy-phenyl-azo)-6-methyl-pyridin-N-oxid der Formel: 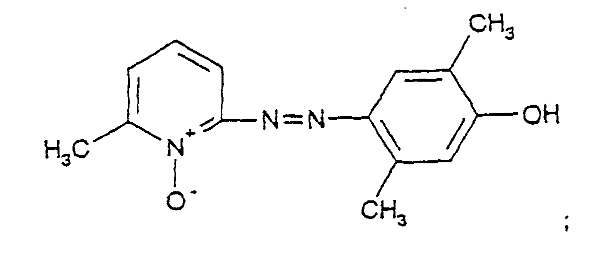
- 2-(4'-Di(β-hydroxyethyl)amino-phenyl-azo)-4-methyl-pyridin-N-oxid der Formel: 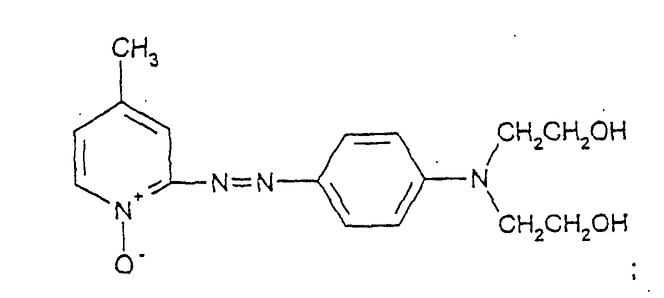
- 2-(4'-Diethylamino-phenyl-azo)-4-methyl-pyridin-N-oxid der Formel: 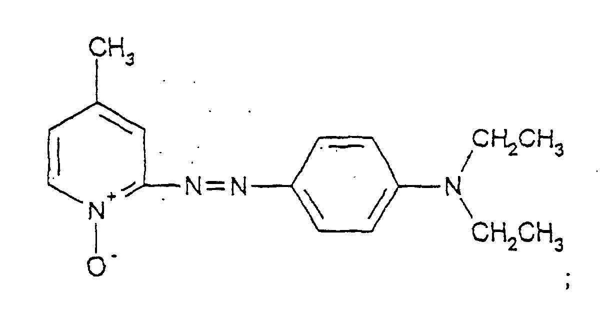
- 2-(4'-Amino-naphthalin-1'-azo)-4-methyl-pyridin-N-oxid der Formel: 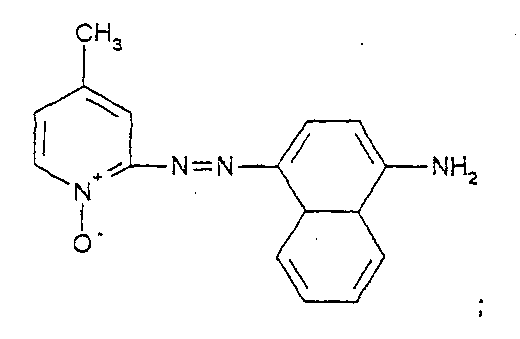
- 2-(2',4'-Diamino-5'-methyl-phenyl-azo)-4-methyl-pyridin-N-oxid der Formel: 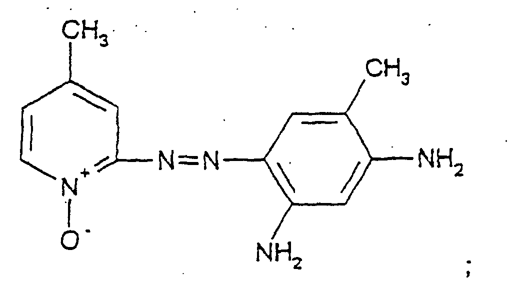
- 2-(4'-Dimethylamino-phenyl-azo)-4-methyl-pyridin-N-oxid der Formel: 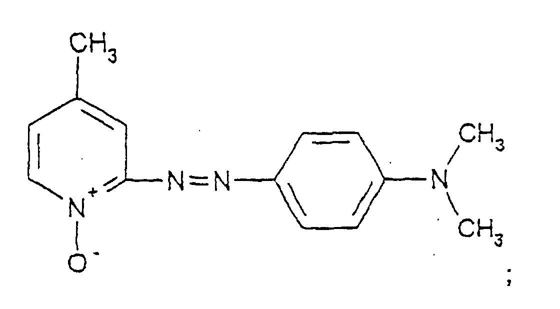
- 2-(4'-Dimethylamino-phenyl-azo)-4,6-dimethyl-pyridin-N-oxid der Formel: 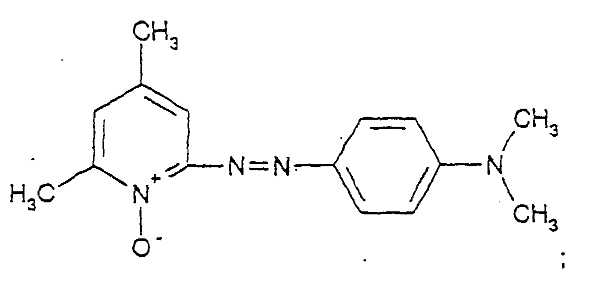
- 2 - (2',5'-Dimethyl-4'-hydroxy-phenyl-azo)-4-methyl-pyridin-N-oxid der Formel: 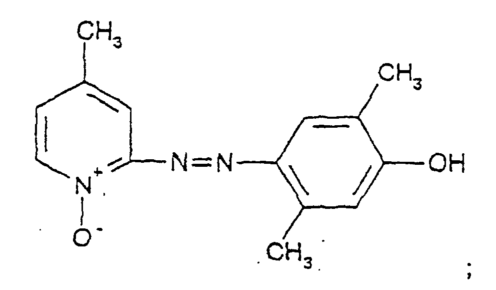
- 2-(4'-Dimethylamino-phenyl-azo)-5-chlor-pyridin-N-oxid der Formel: 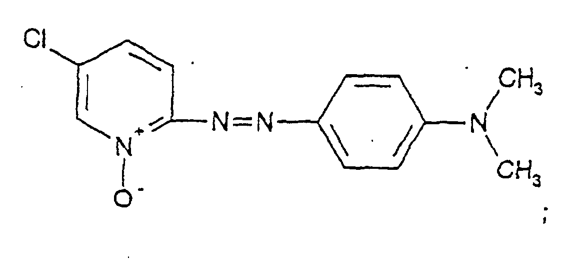
- 2-(4'-Dimethylamino-2'-methyl-phenyl-azo)-5-chlor-pyridin-N-oxid der Formel: 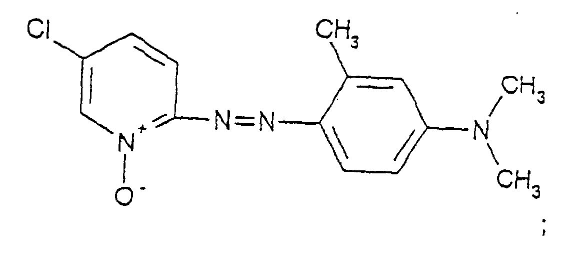
- 2-(4'-Diethylamino-phenyl-azo)-5-chlor-pyridin-N-oxid der Formel: 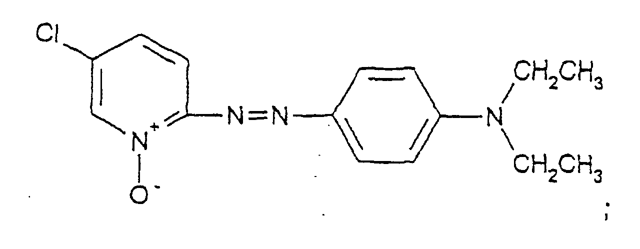
- 2-(6'-Hydroxy-benzomorpholin-7'-azo)-pyridin-N-oxid der Formel: 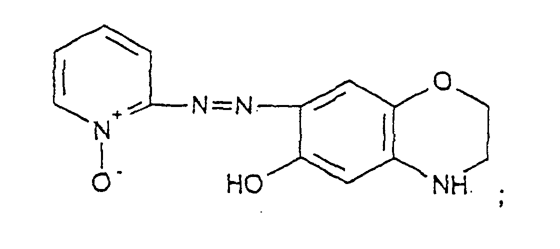
- 2-(6'-Hydroxy-benzomorpholin-7'-azo)-1-methoxy-pyridiniummethosulfat der Formel: 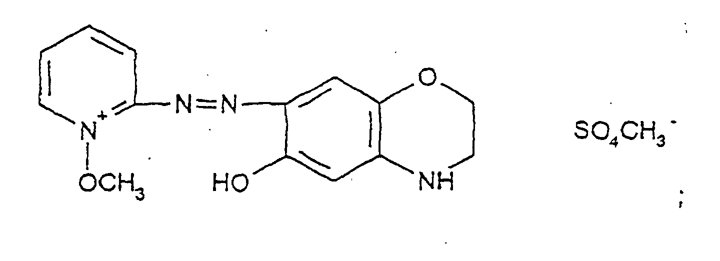
- 2-(2',4'-Diamino-5'-methyl-phenyl-azo)- pyridin-N-oxid der Formel: 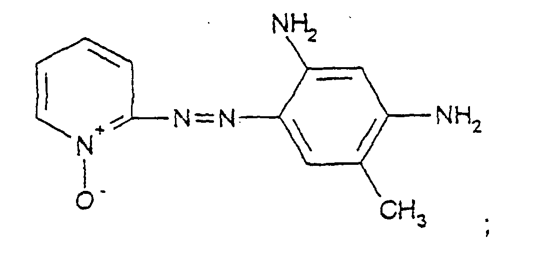
- 2-(2'-Methyl-5'-oxo-4'-phenyl-dihydropyrazolyl-azo)-1-methoxypyridiniummethosulfat der Formel: 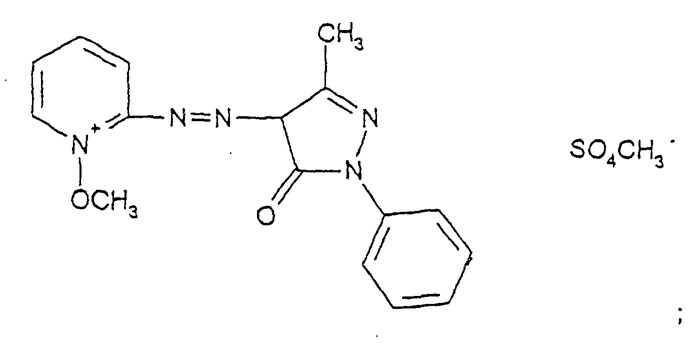
- 2-(3'-Methyl-2',4',6'-trioxo-hexahydro-pyrimidinyl-azo)- pyridin-N-oxid der Formel: 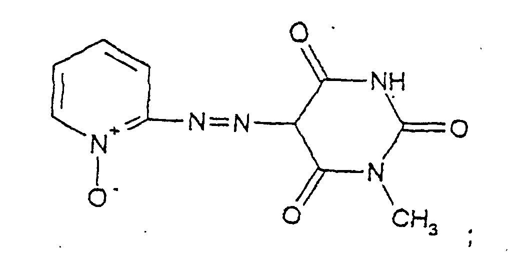
- 2-(4'-Amino-phenyl-azo)-pyridin-N-oxid der Formel: 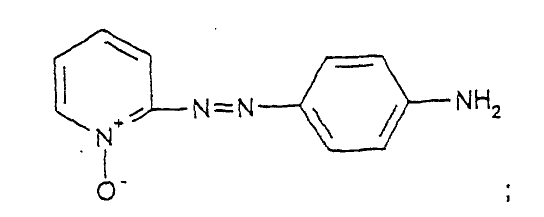
- 2-(4'-(β-Hydroxyethyl)amino-phenyl-azo)-pyridin-N-oxid der Formel: 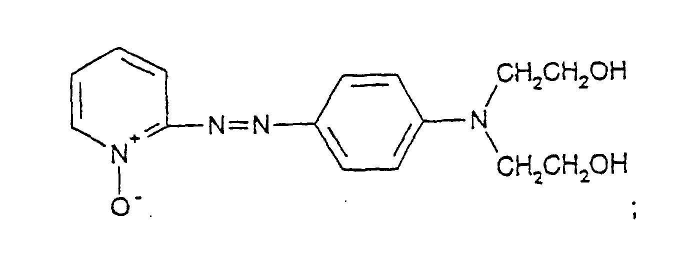
- 2-(4'-(N-Phenylamino)-phenyl-azo)-pyridin-N-oxid der Formel: 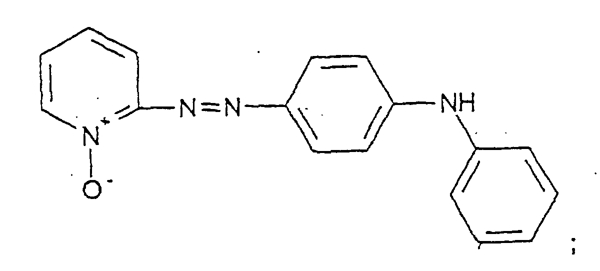
- 2-(4'-(N-Phenylamino)-phenyl-azo)-3-methyl-pyridin-N-oxid der Formel: 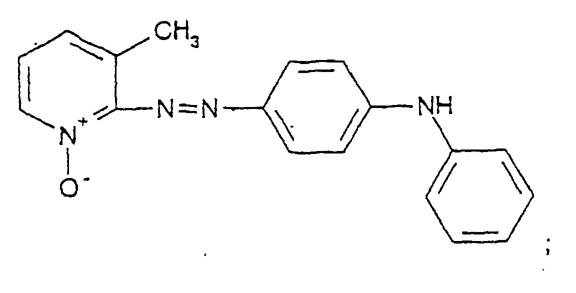
- 2-(4'-Amino-phenyl-azo)-1,3-dimethyl-pyridiniummethosulfat der Formel: 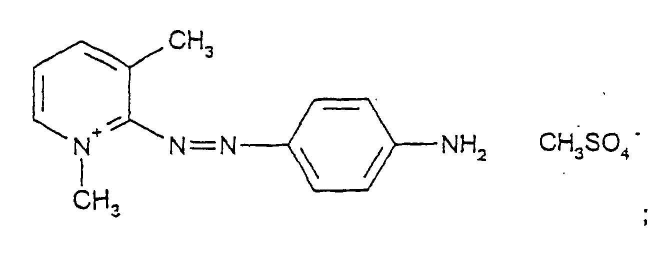
- 2-(4'-(β-Hydroxyethyl)amino-phenyl-azo)-1,4-dimethylpyridiniummethosulfat der Formel: 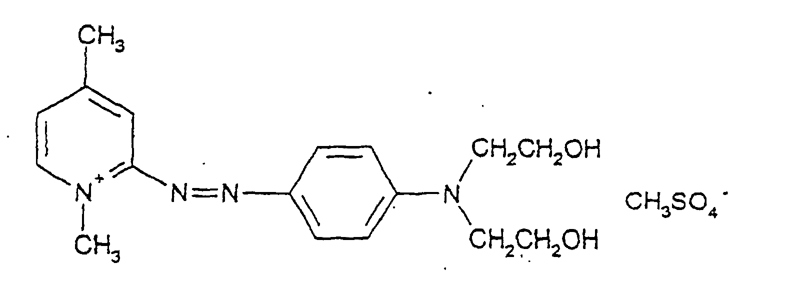
- 2-(4'-Dimethylamino-phenyl-azo)-1,3-dimethylpyridiniummethosulfat der Formel: 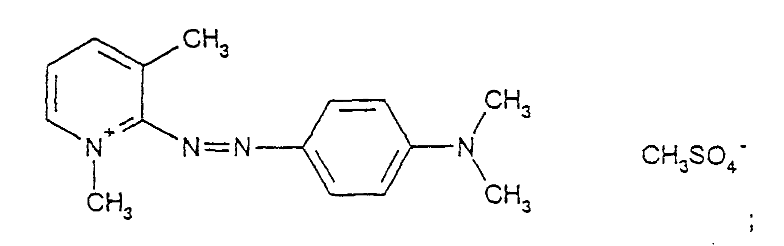
- 2-(4'-N-Phenylamino-phenyl-azo)-1,6-dimethylpyridiniummethosulfat der Formel: 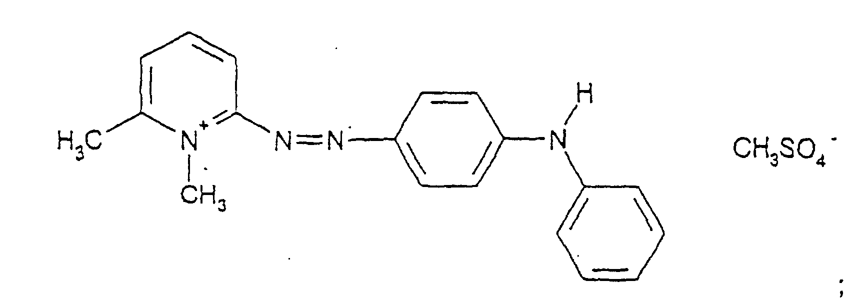
- 2-(4'-Dimethylamino-phenyl-azo)-1,5-dimethylpyridiniummethosulfat der Formel: 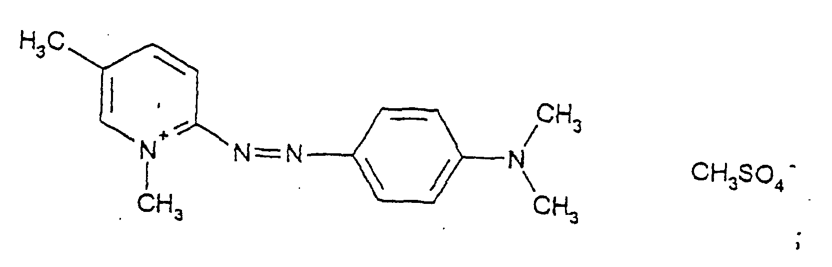
- 2-(4'-Dimethylamino-phenyl-azo)-1,6-dimethylpyridiniummethosulfat der Formel: 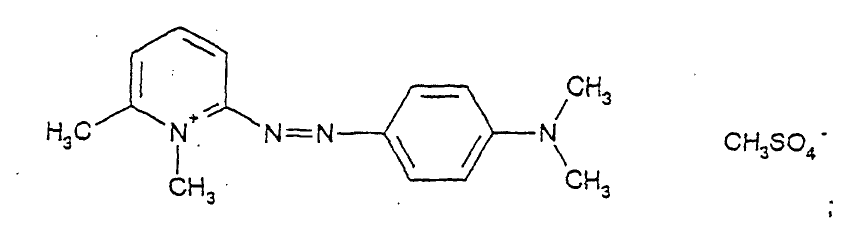
- 2-(2',4',6'-Trioxo-hexahydro-pyrimidinyl-azo)- pyridin-N-oxid der Formel: 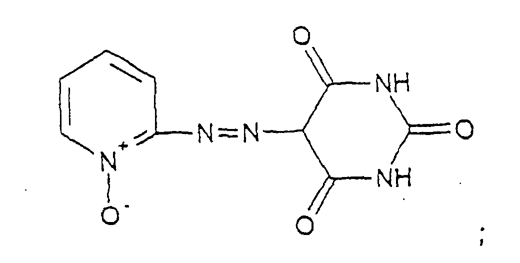
- 2-(4'-Dimethylamino-phenyl-azo)-1,3-dimethyl-benzimidazoliumperchlorat der Formel: 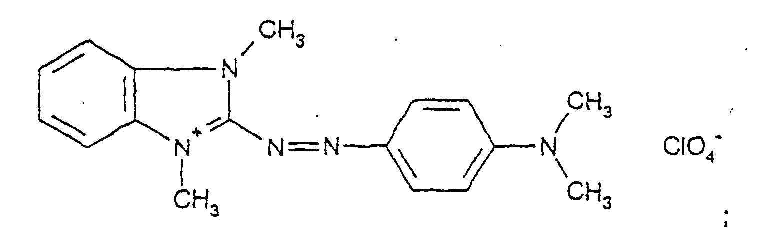
- 2-(2',4'-Diamino-pyridin-3'-azo)-pyridin-N-oxid der Formel:

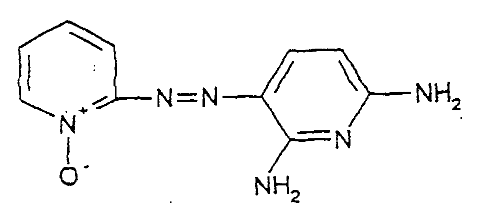

16. Zusammensetzung nach einem der vorhergehenden Ansprüche, **dadurch gekennzeichnet, daß** der oder die kationischen Direktfarbstoffe der Formel (I) 0,001 bis 10 Gew.-% des Gesamtgewichts der Farbmittelzusammensetzung ausmachen.

17. Zusammensetzung nach Anspruch 16, **dadurch gekennzeichnet, daß** der oder die kationischen Direktfarbstoffe der Formel (I) 0,01 bis 5 Gew.-% des Gesamtgewichts der Farbmittelzusammensetzung ausmachen.

18. Zusammensetzung nach einem der vorhergehenden Ansprüche, **dadurch gekennzeichnet, daß** sie einen oder mehrere Kuppler und/oder einen oder mehrere zusätzliche Direktfarbstoffe enthält, die von den in Anspruch 1 definierten Direktfarbstoffen verschieden sind.

19. Zusammensetzung nach Anspruch 18, **dadurch gekennzeichnet, daß** der oder die Kuppler unter den m-Phenylendiaminen, m-Aminophenolen, m-Dihydroxybenzolen und heterocyclischen Kupplern und deren Additionssalzen mit einer Säure ausgewählt sind.

20. Zusammensetzung nach Anspruch 18 oder 19, **dadurch gekennzeichnet, daß** der oder die Kuppler 0,0001 bis 10 Gew.-% des Gesamtgewichts der Farbmittelzusammensetzung ausmachen.

21. Zusammensetzung nach Anspruch 20, **dadurch gekennzeichnet, daß** der oder die Kuppler 0,005 bis 5 Gew.-% des Gesamtgewichts der Farbmittelzusammensetzung ausmachen.

22. Zusammensetzung nach einem der vorhergehenden Ansprüche, **dadurch gekennzeichnet, daß** die Additionssalze mit einer Säure unter den Hydrochloriden, Hydrobromiden, Sulfaten, Tartraten, Lactaten und Acetaten ausgewählt sind.

23. Verfahren zum Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 1 bis 22 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

24. Verfahren nach Anspruch 23, **dadurch gekennzeichnet, daß** das in der oxidierenden Zusammensetzung vorliegende Oxidationsmittel unter Wasserstoffperoxid, Harnstoffperoxid, Alkalimetallbromaten, Salzen von Persäuren, wie Perboraten, Percarbonaten und Persulfaten, Persäuren und Enzymen ausgewählt ist.

25. Vorrichtung mit mehreren Abteilungen oder "Kit" zum Färben mit mehreren Abteilungen, wobei eine Abteilung eine Farbmittelzusämmensetzung nach einem der Ansprüche 1 bis 22 und eine zweite Abteilung eine oxidierende Zusammensetzung enthält.

## Claims

1. Composition for the oxidation dyeing of keratin fibres, and in particular human keratin fibres such as the hair, **characterized in that** it comprises, in a medium which is suitable for dyeing:
- at least one oxidation base,
- at least one cationic direct dye of formula (I) below:
A―N=N―B (I)
in which:
A represents a group chosen from structures A1 to A3 below: 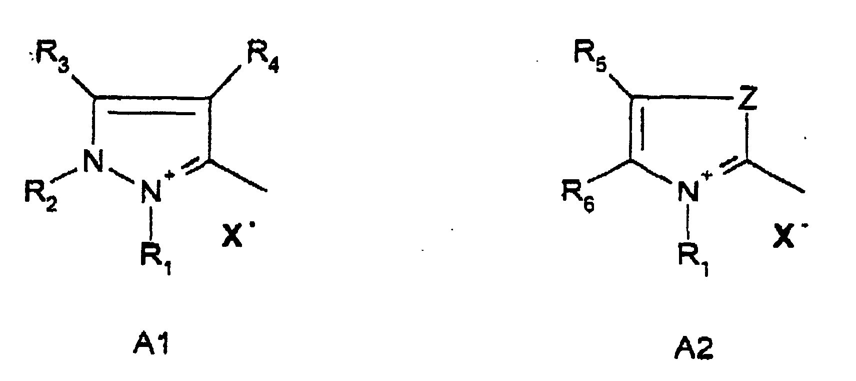 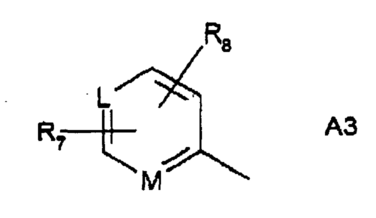 in which structures A1 to A3:
- R₁ denotes a C₁-C₄ alkyl radical, a phenyl radical or a phenyl radical substituted with a C₁-C₄ alkyl radical or with a halogen atom chosen from chlorine, bromine, iodine and fluorine;
- R₂ denotes a C₁-C₄ alkyl radical or a phenyl radical;
- R₃ and R₄, which may be identical or different, represent a C₁-C₄ alkyl radical, a phenyl radical or together form a benzene ring substituted with one or more C₁-C₄ alkyl, C₁-C₄ alkoxy or nitro radicals; R₃ can also denote a hydrogen atom;
- R₅ and R₆, which may be identical or different, represent a C₁-C₄ alkyl radical, a phenyl radical or together form a benzene ring which is unsubstituted or substituted with one or more C₁-C₄ alkyl, C₁-C₄ alkoxy or nitro radicals; R₅ can also denote a hydrogen atom;
- R₇ and R₈, which may be identical or different, represent a hydrogen atom or a halogen atom chosen from chlorine, bromine, iodine and fluorine, a C₁-C₄ alkyl or C₁-C₄ alkoxy radical or a nitro radical;
- Z denotes an oxygen or sulphur atom or a group NR₂ in which R₂ can take the same meaning as that given above;
- the ring member L represents a group -CH, -CR or -N⁺R₉(X⁻)ᵣ;
- the ring member M represents a group -CH, -CR or -N⁺R₉(X⁻)ᵣ;
- r is an integer equal to 0 or 1;
- R represents a C₁-C₄ alkyl radical;
- R₉ represents an atom O⁻ or a C₁-C₄ alkyl or C₁-C₄ alkoxy radical;
- X⁻ represents an anion;
it being understood that:
- in the groups of formula A3, at least one of the ring members L and M represents a group -N⁺R₉(X⁻)ᵣ;
- when R₆ represents a C₁-C₄ alkyl radical and Z represents a sulphur atom, then R₅ is other than a C₁-C₄ alkyl radical;
- when R₆ represents a C₁-C₄ alkyl radical and Z represents a sulphur atom, and when one of the radicals R₁₀ and R₁₁ represents a hydrogen atom, then R₅ is other than a hydrogen atom;
- when R₉ denotes O⁻, then r = 0;
- when L or M denotes a radical -N⁺R₉(X⁻)ᵣ in which R₉ represents a C₁-C₄ alkyl radical and r = 1, then at least one of the radicals R₇ and R₈ is other than a hydrogen atom;
- when L denotes a radical -N⁺R₉(X⁻)ᵣ, then M represents a group -CH or CR;
- when M denotes a radical -NR₉(X⁻)ᵣ, then L represents a group -CH or CR;
- when Z represents a group NR₂ in which R₂ represents a C₁-C₄ alkyl radical, then at least one of the radicals R₁, R₅ and R₆ is other than a C₁-C₄ alkyl radical;
- when Z represents a sulphur atom and R₁ represents a C₁-C₄ alkyl radical, and when one of the radicals R₁₀ and R₁₁ represents a hydrogen atom, then R₅ and R₆ cannot together form an unsubstituted benzene ring;
B represents:
either (a) a group of structure B1 below: 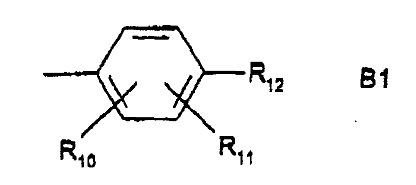 in which structure B1,
- R₁₀ represents a hydrogen atom, a halogen atom chosen from chlorine, bromine, iodine and fluorine, a C₁-C₄ alkyl, C₁-C₄ alkoxy or hydroxyl radical, a group -NHR₁₃, -NR₁₄R₁₅, -NHCO(C₁-C₄)alkyl or nitro, or forms with R₁₁ a 5- or 6-membered carbon ring or a ring containing one or more hetero atoms chosen from nitrogen, oxygen and sulphur;
- R₁₁ represents a hydrogen atom, a halogen atom chosen from chlorine, bromine, iodine and fluorine, a C₁-C₄ alkyl or C₁-C₄ alkoxy radical or forms with R₁₂ or R₁₃ a 5- or 6-membered carbon ring or a ring containing one or more hetero atoms chosen from nitrogen, oxygen and sulphur;
- R₁₂ represents a hydrogen atom, a hydroxyl radical, a radical -NHR₁₃ or a radical -NR₁₄R₁₅;
- R₁₃ represents a hydrogen atom or a C₁-C₄ alkyl, C₁-C₄ monohydroxyalkyl, C₂-C₄ polyhydroxyalkyl or phenyl radical;
- R₁₄ and R₁₅, which may be identical or different, represent a C₁-C₄ alkyl, C₁-C₄ monohydroxyalkyl or C₂-C₄ polyhydroxyalkyl radical;
or (b) a 5- or 6-membered nitrogenous heterocyclic group, a 5- or 6-membered nitrogenous heterocyclic group containing one or more hetero atoms chosen from oxygen and sulphur and/or one or more carbonyl groups, it being possible for the said heterocycles to be substituted with one or more C₁-C₄ alkyl, amino or phenyl radicals.

2. Composition according to Claim 1, **characterized in that** the anion X⁻ is chosen from chloride, methyl sulphate, ethyl sulphate, acetate and perchlorate.

3. Composition according to Claim 1 or 2, **characterized in that** the 5- or 6-membered nitrogenous heterocyclic groups are chosen from the groups of structure B2 below: 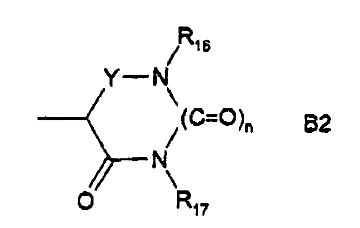 in which structure B2:
- R₁₆ and R₁₇, which may be identical or different, represent a hydrogen atom, a C₁-C₄ alkyl radical or a phenyl radical;
- Y represents a group 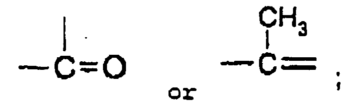
- n is an integer equal to 0 or 1.

4. Composition according to any one of the preceding claims, **characterized in that** the oxidation base(s) is (are) chosen from para-phenylenediamines, double bases, para-aminophenols, ortho-aminophenols and heterocyclic oxidation bases.

5. Composition according to Claim 4, **characterized in that** the para-phenylenediamines are chosen from the compounds of formula (II) below, and the addition salts thereof with an acid: 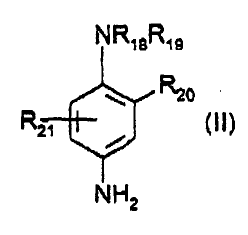 in which:
- R₁₈ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical, a C₁-C₄ alkyl radical substituted with a nitrogenous group, a phenyl group or a 4'-aminophenyl group;
- R₁₉ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
- R₂₀ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino(C₁-C₄)alkoxy radical, a C₁-C₄ mesylaminoalkoxy radical or a carbamoylamino(C₁-C₄)alkoxy radical,
- R₂₁ represents a hydrogen or halogen atom or a C₁-C₄ alkyl radical.

6. Composition according to Claim 5, **characterized in that** the para-phenylenediamines of formula (II) are chosen from para-phenylenediamine, paratolylenediamine, 2-chloro-para-phenylenediamine, 2,3-dimethyl-para-phenylenediamine, 2,6-dimethyl-paraphenylenediamine, 2,6-diethyl-para-phenylenediamine, 2,5-dimethyl-para-phenylenediamine, N,N-dimethyl-paraphenylenediamine, N,N-diethyl-para-phenylenediamine, N,N-dipropyl-para-phenylenediamine, 4-amino-N,N-diethyl-3-methylaniline, N,N-bis(β-hydroxyethyl)-paraphenylenediamine, 4-amino-N,N-bis(β-hydroxyethyl)-2-methylaniline, 4-amino-2-chloro-N,N-bis(β-hydroxyethyl)aniline, 2-β-hydroxyethyl-paraphenylenediamine, 2-fluoro-para-phenylenediamine, 2-isopropyl-para-phenylenediamine, N- (β-hydroxypropyl)-para-phenylenediamine, 2-hydroxymethyl-paraphenylenediamine, N,N-dimethyl-3-methyl-paraphenylenediamine, N,N-(ethyl-β-hydroxyethyl)-paraphenylenediamine, N-(β,γ-dihydroxypropyl)-paraphenylenediamine, N-(4'-aminophenyl) -para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

7. Composition according to Claim 4, **characterized in that** the double bases are chosen from the compounds of formula (III) below, and the addition salts thereof with an acid: 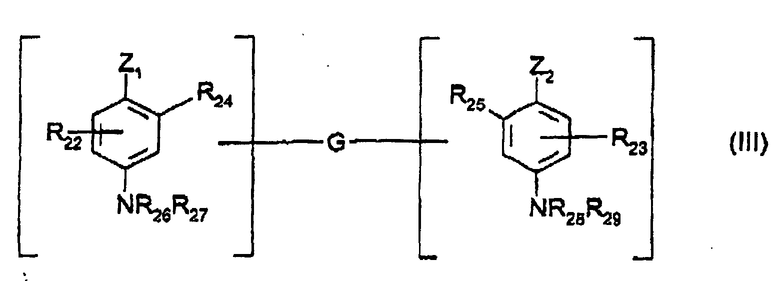 in which:
- Z₁ and Z₂, which may be identical or different, represent a hydroxyl or -NH₂ radical which may be substituted with a C₁-C₄ alkyl radical or with a linker arm G;
- the linker arm G represents a linear or branched alkylene chain containing from 1 to 14 carbon atoms, which may be interrupted by or terminated with one or more nitrogenous groups and/or one or more hetero atoms such as oxygen, sulphur or nitrogen atoms, and optionally substituted with one or more hydroxyl or C₁-C₆ alkoxy radicals;
- R₂₂ and R₂₃ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm G;
- R₂₄, R₂₅, R₂₆, R₂₇, R₂₈ and R₂₉, which may be identical or different, represent a hydrogen atom, a linker arm G or a C₁-C₄ alkyl radical;
it being understood that the compounds of formula (III) contain only one linker arm G per molecule.

8. Composition according to Claim 7, **characterized in that** the double bases of formula (III) are chosen from N,N'-bis(β-hydroxyethyl)-N,N-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)-tetramethylenediamine, N,N'-bis(4-methylaminophenyl)tetramethylenediamine, N,N'-bis-(ethyl)-N,N'-bis(4'-amino-3'-methylphenyl)ethylenediamine and 1,8-bis(2,5-diaminophenoxy)-3,5-dioxaoctane, and the addition salts thereof with an acid.

9. Composition according to Claim 4, **characterized in that** the para-aminophenols are chosen from the compounds of formula (IV) below, and the addition salts thereof with an acid: 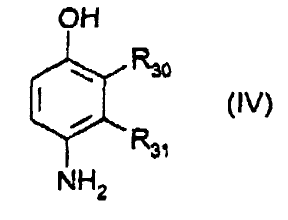 in which:
- R₃₀ represents a hydrogen or halogen atom or a C₁-C₄ alkyl, C₁-C₄ monohydroxyalkyl, (C₁-C₄) alkoxy(C₁-C₄)alkyl, C₁-C₄ aminoalkyl or hydroxy (C₁-C₄) alkylamino (C₁-C₄) alkyl radical,
- R₃₁ represents a hydrogen or halogen atom or a C₁-C₄-alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy-(C₁-C₄)alkyl radical,
it being understood that at least one of the radicals R₃₀ or R₃₁ represents a hydrogen atom.

10. Composition according to Claim 9, **characterized in that** the para-aminophenols of formula (IV) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

11. Composition according to Claim 4, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

12. Composition according to Claim 4, **characterized in that** the heterocyclic oxidation bases are chosen from pyridine derivatives, pyrimidine derivatives and pyrazole derivatives, and the addition salts thereof with an acid.

13. Composition according to any one of the preceding claims, **characterized in that** the oxidation base(s) represent(s) from 0.0005 to 12% by weight relative to the total weight of the dye composition.

14. Composition according to Claim 13, **characterized in that** the oxidation base(s) represent(s) from 0.005 to 6% by weight relative to the total weight of the dye composition.

15. Composition according to any one of the preceding claims, **characterized in that** the cationic direct dye(s) of formula (I) is (are) chosen from:
- 4'-dimethylaminophenyl-2-azopyridine N-oxide of formula: 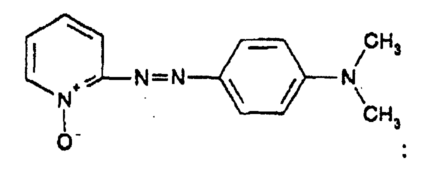
- 4'-dimethylamino-2'-methylphenyl-2-azopyridine N-oxide of formula: 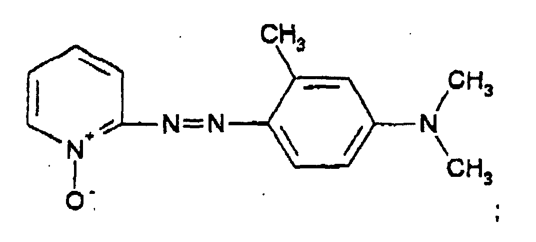
- 2-[(1,3-diamino-6-methyl-4-phenyl)azo] -3-methylbenzothiazolium iodide of formula: 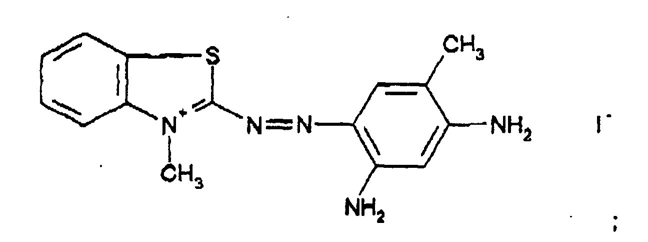
- 6'-hydroxybenzomorpholine-7'-aza-2-methyl-3-benzothiazolium chloride of formula: 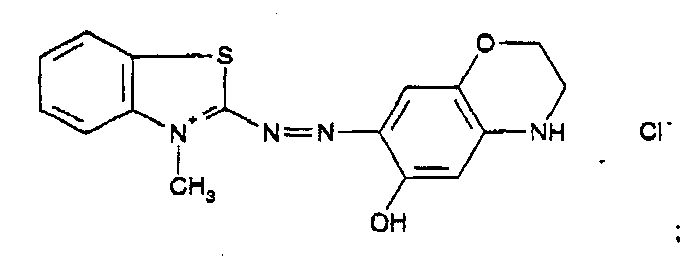
- 6'-hydroxybenzomorpholine-7'-aza-2-methyl-4-phenyl-3-thiazolium perchlorate of formula: 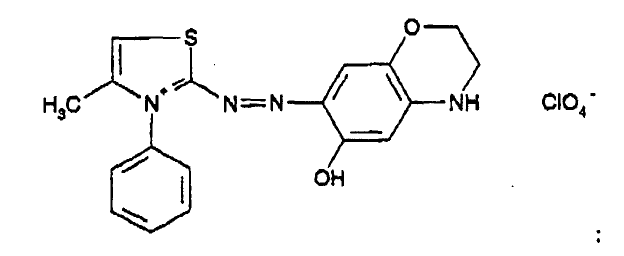
- 6'-hydroxybenzomorpholine-7'-azo-2-diphenyl-3,4-thiazolium perchlorate of formula: 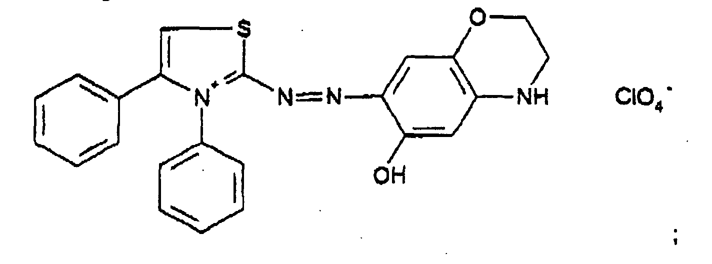
- 6'-hydroxybenzomorpholine-7'-aza-2-methyl-3-benzothiazolium chloride of formula: 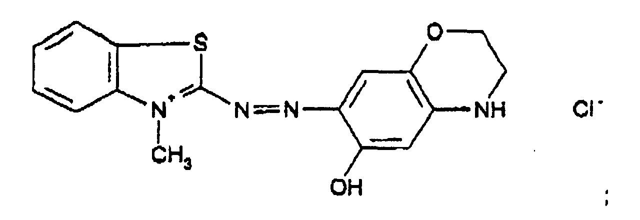
- 6'-hydroxybenzomorpholine-7'-aza-2-methyl-4-phenyl-3-thiazolium perchlorate of formula: 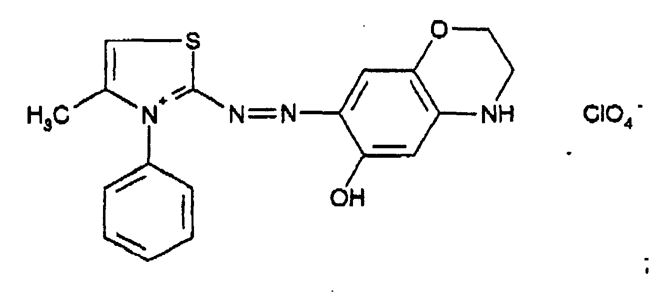
- 2',4'-diamino-5'-methoxyphenyl-2-azopyridine N-oxide of formula: 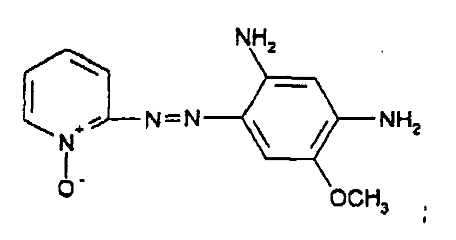
- 2'-acetamido-4'-hydroxy-5'-methylphenyl-2-azopyridine N-oxide of formula: 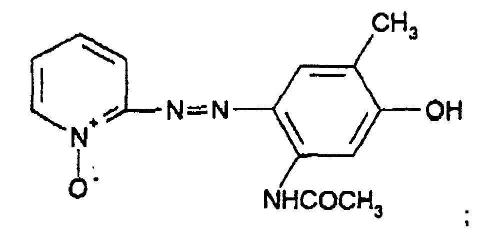
- 2'-acetamido-4'-dimethylaminophenyl-2-azopyridine N-oxide of formula: 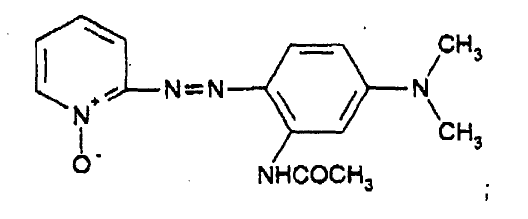
- 2'-amino-4'-dimethylaminophenyl-2-azopyridine N-oxide of formula: 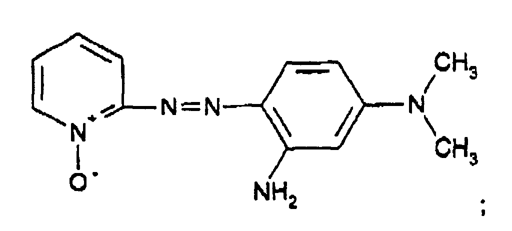
- 2',4'-diamino-5'-methylphenyl-2-azomethoxy-1-pyridinium perchlorate of formula: 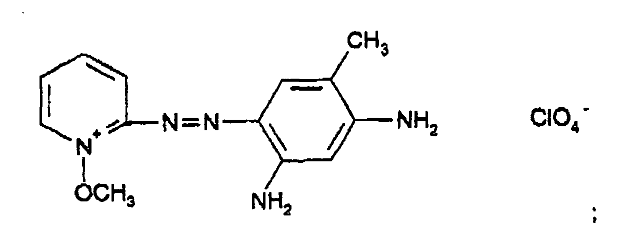
- 2',5'-dimethyl-4'-hydroxyphenyl-2-azopyridine N-oxide of formula: 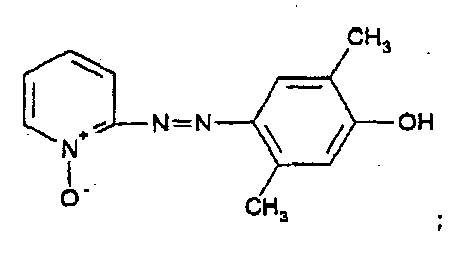
- 2'-dimethylamino-4'-hydroxyphenyl-2-azopyridine N-oxide of formula: 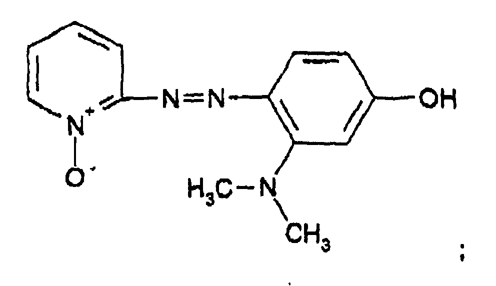
- 4'-dimethylamino-2'-hydroxyphenyl-2-azopyridine N-oxide of formula: 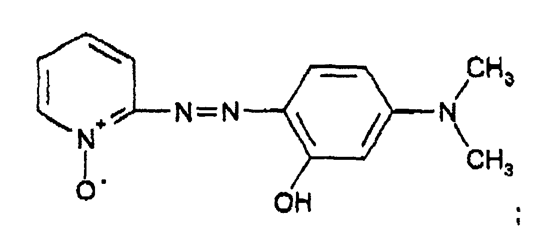
- 3',5'-dimethyl-4'-hydroxyphenyl-2-azopyridine N-oxide of formula: 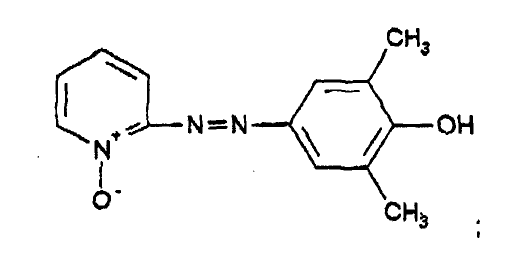
- 4'-dimethylamino-2'-nitrophenyl-2-azopyridine N-oxide of formula: 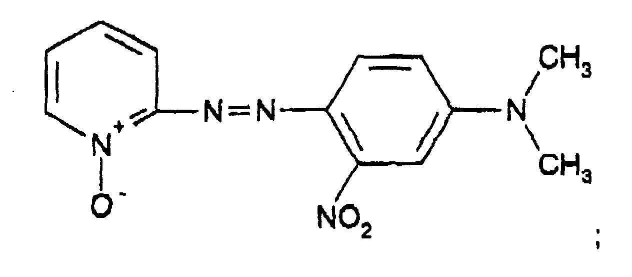
- 8'-hydroxyquinoline-5':2'-azopyridine N-oxide of formula: 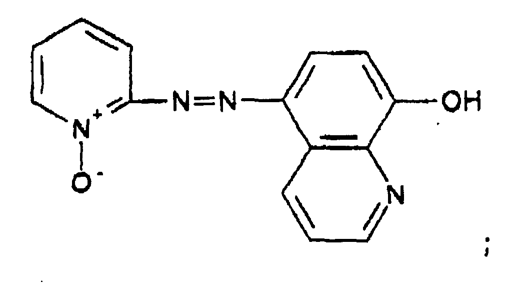
- 2'-nitro-4'-dimethylaminophenyl-2-azomethoxy-1-pyridinium methosulphate of formula: 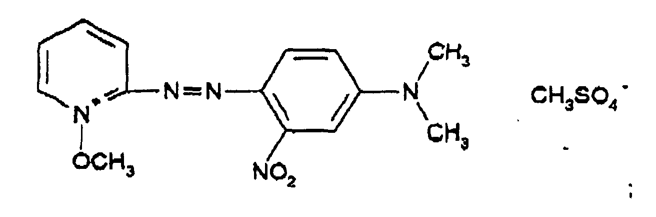
- 4'-dimethylaminophenyl-2-azo-5-nitropyridine N-oxide of formula: 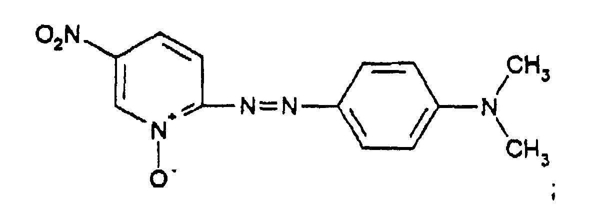
- 4'-dimethylaminophenyl-2-azo-6-methylpyridine N-oxide of formula: 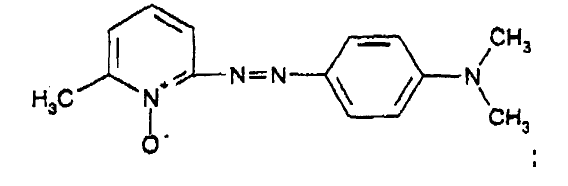
- 4'-dimethylaminophenyl-2-azo-5-methylpyridine N-oxide of formula: 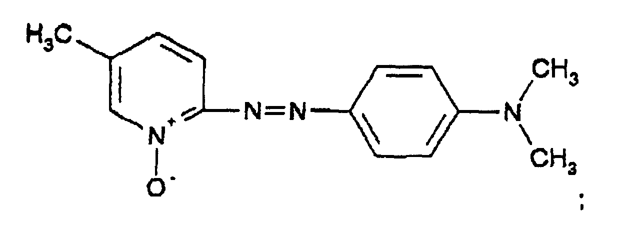
- 4'-dimethylaminophenyl-2-azo-3-methylpyridine N-oxide of formula: 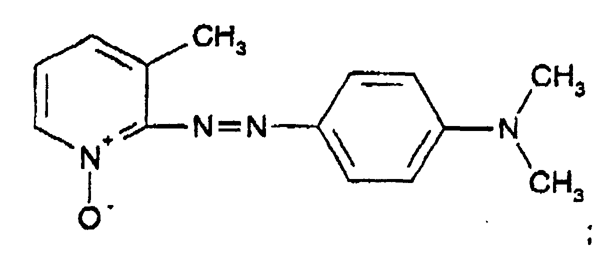
- 4'-diethylaminophenyl-2-azo-3-methylpyridine N-oxide of formula: 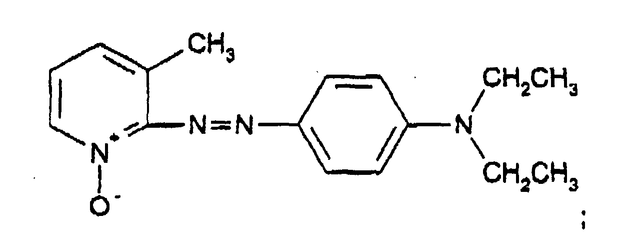
- 4'-dimethylamino-2'-acetylaminophenyl-2-azo-3-methylpyridine N-oxide of formula: 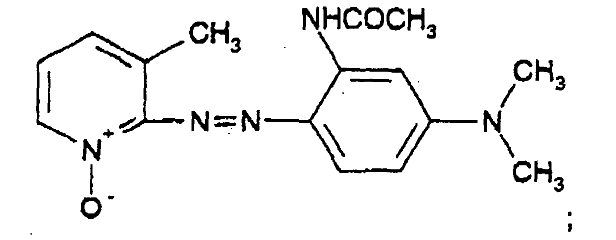
- 4'-aminonaphthalene-1':2-azo-6-methylpyridine N-oxide of formula: 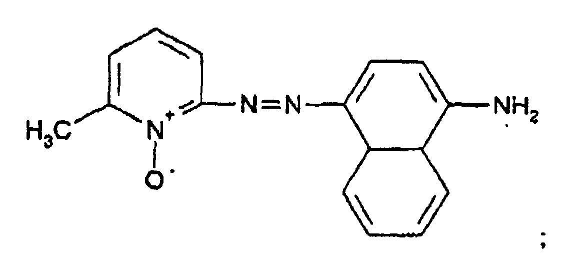
- 4'-di(β-hydroxyethyl)aminophenyl-2-azo-6-methylpyridine N-oxide of formula: 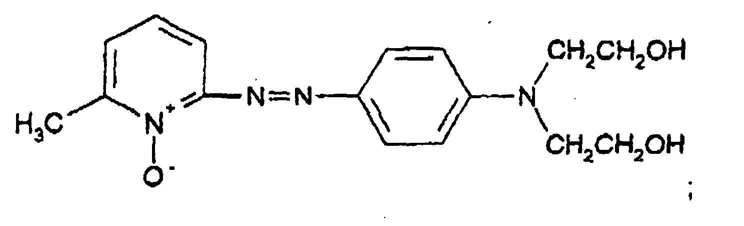
- 4'-diethylaminophenyl-2-azo-6-methylpyridine N-oxide of formula: 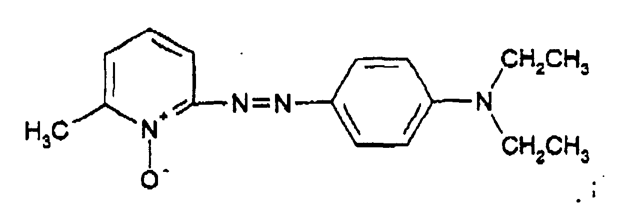
- 2',5'-dimethyl-4'-hydroxyphenyl-2-azo-6-methylpyridine N-oxide of formula : 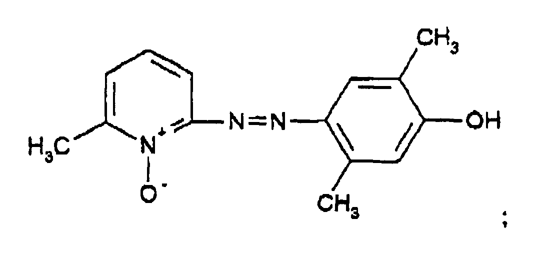
- 4'-di(β-hydroxyethyl)aminophenyl-2-azo-4-methylpyridine N-oxide of formula: 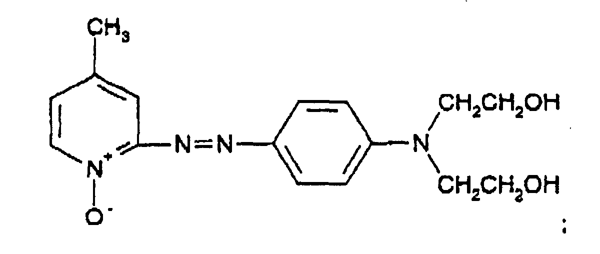
- 4'-diethylaminophenyl-2-azo-4-methylpyridine N-oxide of formula: 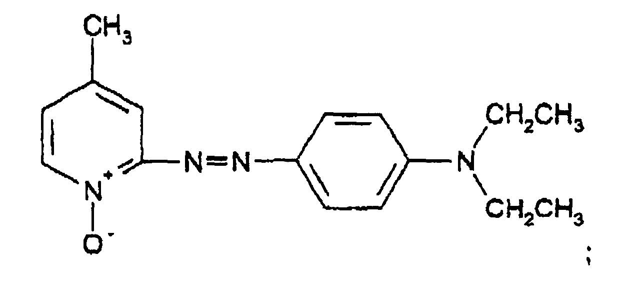
- 4'-aminonaphthalene-1':2-azo-4-methylpyridine N-oxide of formula: 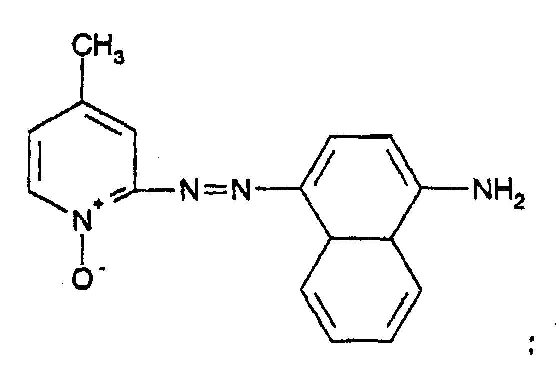
- 2',4'-diamino-5'-methylphenyl-2-azo-2-4-methylpyridine N-oxide of formula: 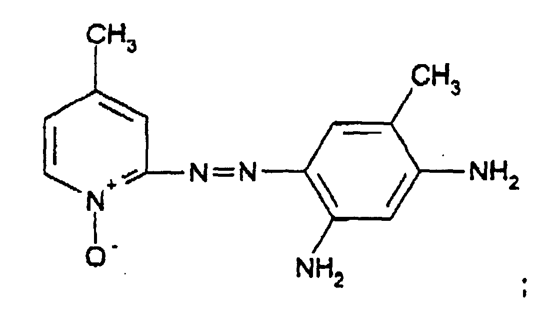
- 4'-dimethylaminophenyl-2-azo-4-methylpyridine N-oxide of formula: 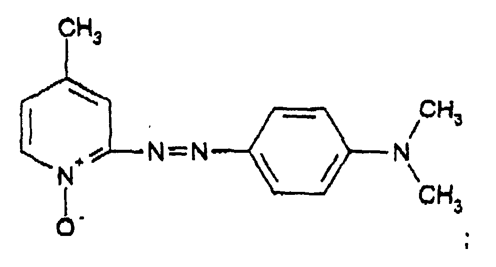
- 4'-dimethylaminophenyl-2-azo-4,6-dimethylpyridine N-oxide of formula: 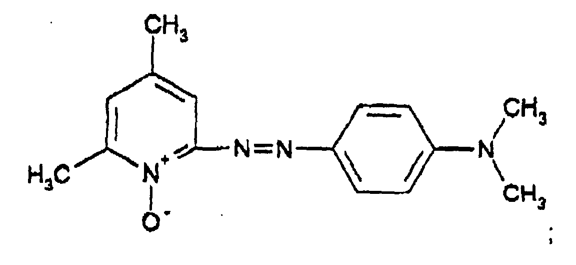
- 2',5'-dimethyl-4'-hydroxyphenyl-2-azo-4-methylpyridine N-oxide of formula: 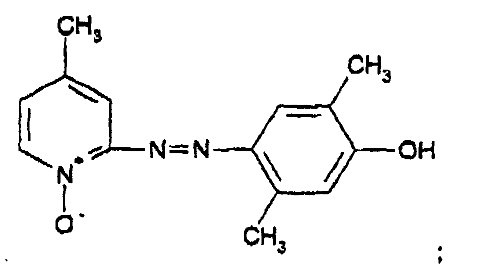
- 4'-dimethylaminophenyl-2-azo-5-chloropyridine N-oxide of formula: 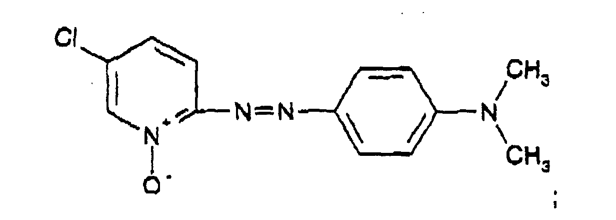
- 4'-dimethylamino-2'-methylphenyl-2-azo-5-chloropyridine N-oxide of formula: 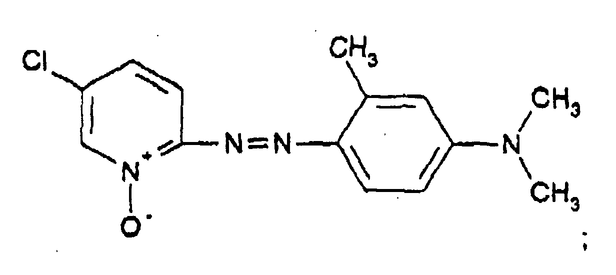
- 4'-diethylaminophenyl-2-azo-5-chloropyridine N-oxide of formula: 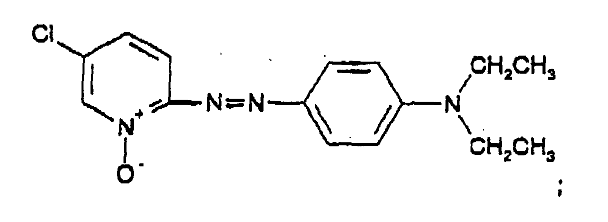
- 6'-hydroxybenzomorpholine-7':2-azopyridine N-oxide of formula: 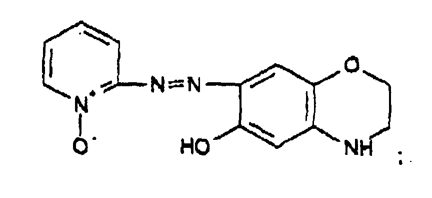
- 6'-hydroxybenzomorpholine-7':2-azo-1-methoxypyridinium methosulphate of formula: 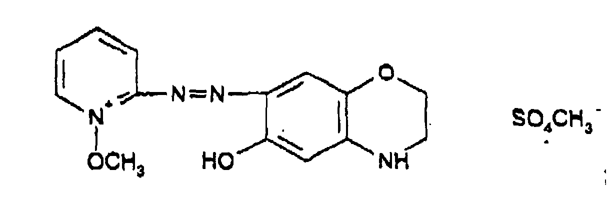
- 2',4'-diamino-5'-methylphenyl-2-azopyridine N-oxide of formula: 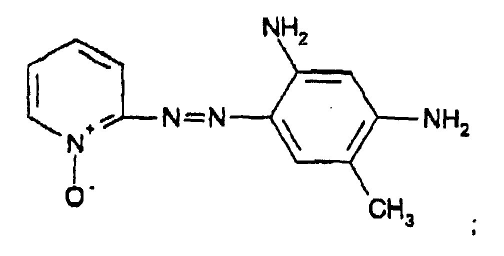
- 2'-methyl-5'-oxo-4'-phenyldihydropyrazolyl-2-azo-1-methoxypyridinium methosulphate of formula: 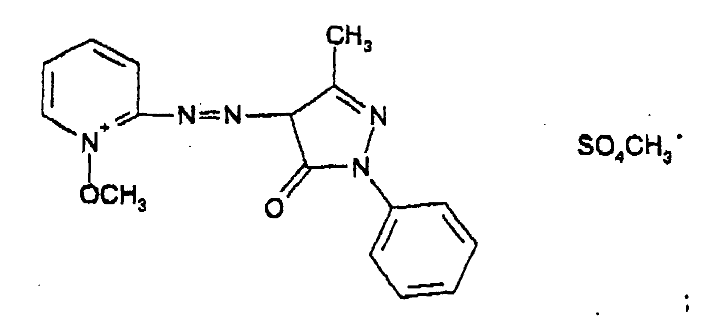
- 3'-methyl-2',4',6'-trioxohexahydropyrimidinyl-2-azopyridine N-oxide of formula: 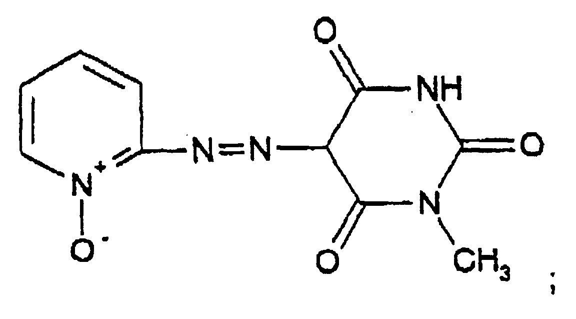
- 4'-aminophenyl-2-azopyridine N-oxide of formula: 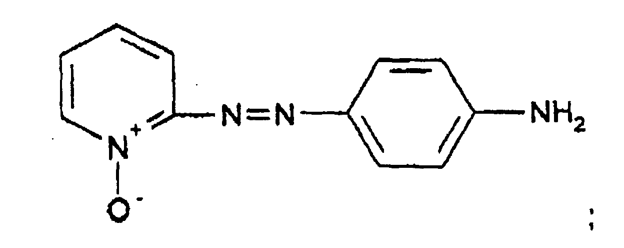
- 4'-di (β-hydroxyethyl) aminophenyl-2-azopyridine N-oxide of formula: 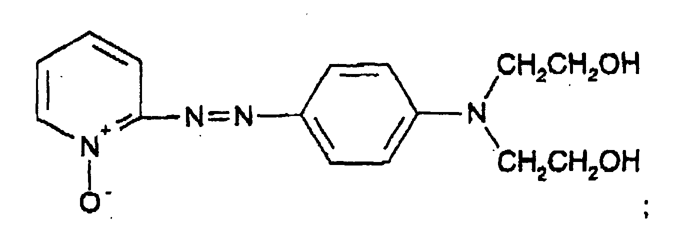
- 4'-(N-phenylamino)phenyl-2-azopyridine N-oxide of formula: 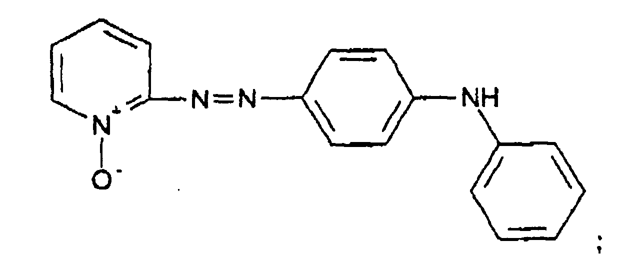
- 4'-(N-phenylamino)phenyl-2-azo-3-methylpyridine N-oxide of formula: 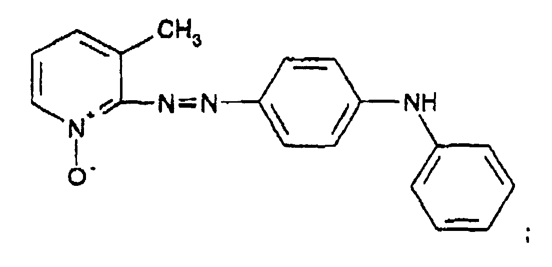
- 4'-aminophenyl-2-azo-1,3-dimethylpyridinium methosulphate of formula: 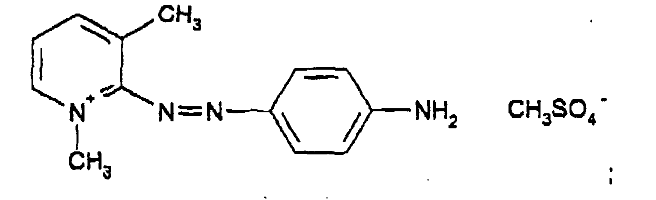
- 4'-di(β-hydroxyethyl)aminophenyl-2-azo-1,4-dimethylpyridinium methosulphate of formula: 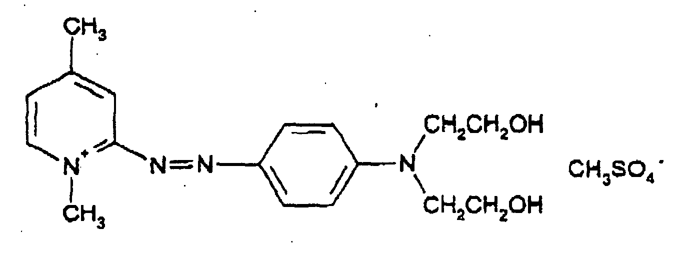
- 4'-dimethylaminophenyl-2-azo-1,3-dimethylpyridinium methosulphate of formula: 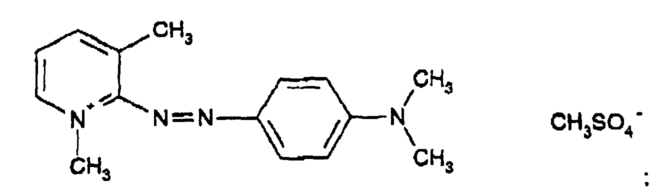
- 4'-N-phenylaminophenyl-2-azo-1,6-dimethylpyridinium methosulphate of formula: 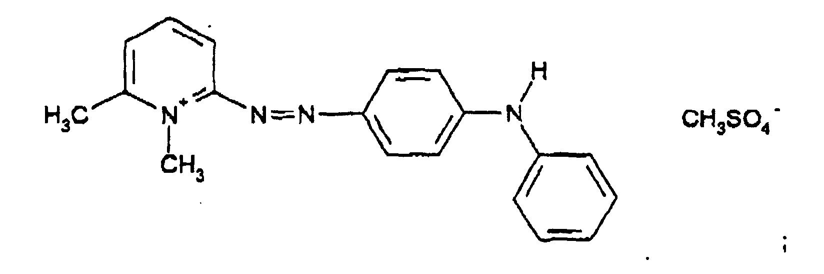
- 4'-dimethylaminophenyl-2-azo-1,5-dimethylpyridinium methosulphate of formula: 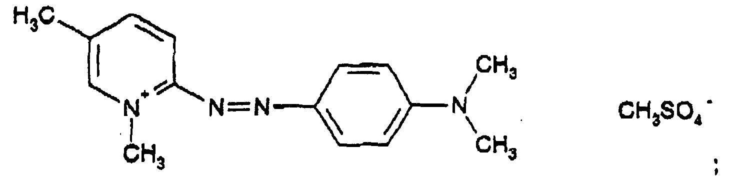
- 4'-dimethylaminophenyl-2-azo-1,6-dimethylpyridinium methosulphate of formula: 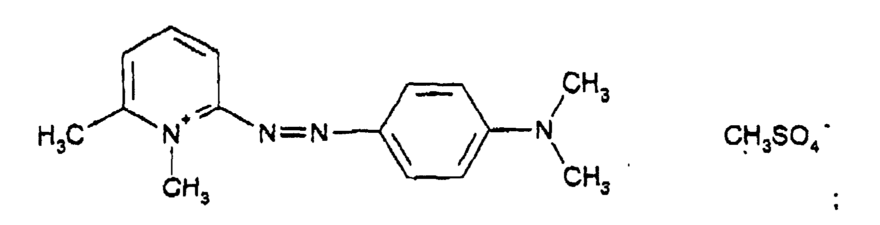
- 2',4',6'-trioxohexahydropyrimidinyl-2-azopyridine N-oxide of formula: 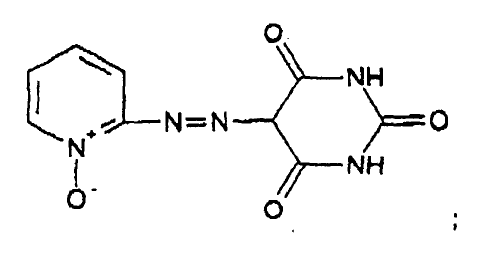
- 4'-dimethylaminophenyl-2-azo-1,3-dimethylbenzimidazolium perchlorate of formula: 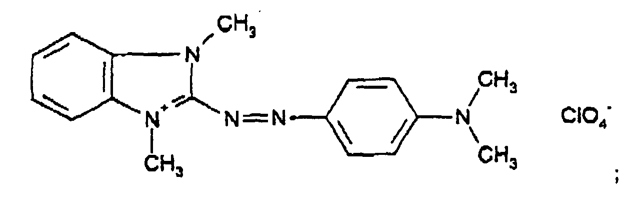 and
- 2',4'-diamino-3'-pyridine-2-azopyridine N-oxide of formula:

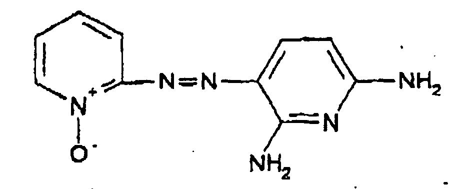

16. Composition according to any one of the preceding claims, **characterized in that** the cationic direct dye(s) of formula (I) represent(s) from 0.001 to 10% by weight relative to the total weight of the dye composition.

17. Composition according to Claim 16, **characterized in that** the cationic direct dye(s) of formula (I) represent(s) from 0.01 to 5% by weight relative to the total weight of the dye composition.

18. Composition according to any one of the preceding claims, **characterized in that** it contains one or more couplers and/or one or more additional direct dyes other than the cationic direct dyes of formula (I) as defined in Claim 1.

19. Composition according to Claim 18, **characterized in that** the coupler(s) is (are) chosen from meta-phenylenediamines, meta-aminophenols, metadiphenols and heterocyclic couplers, and the addition salts thereof with an acid.

20. Composition according to Claim 18 or 19, **characterized in that** the coupler(s) represent(s) from 0.0001 to 10% by weight relative to the total weight of the dye composition.

21. Composition according to Claim 20, **characterized in that** the coupler(s) represent(s) from 0.005 to 5% by weight relative to the total weight of the dye composition.

22. Composition according to any one of the preceding claims, **characterized in that** the addition salts with an acid are chosen from the hydrochlorides, hydrobromides, sulphates, tartrates, lactates and acetates.

23. Process for dyeing keratin fibres, and in particular human keratin fibres such as the hair, **characterized in that** at least one dye composition as defined in any one of Claims 1 to 22 is applied to the said fibres and in that the colour is developed at acidic, neutral or alkaline pH by means of an oxidizing agent which is added to the dye composition just at the time of use, or which is present in an oxidizing composition that is applied simultaneously or sequentially.

24. Process according to Claim 23, **characterized in that** the oxidizing agent present in the oxidizing composition is chosen from hydrogen peroxide, urea peroxide, alkali metal bromates, persalts such as perborates, percarbonates and persulphates, peracids and enzymes.

25. Multi-compartment device or multi-compartment dyeing "kit", a first compartment of which contains a dye composition as defined in any one of Claims 1 to 22 and a second compartment of which contains an oxidizing composition.
